# EUROPEAN PATENT APPLICATION

(11) **EP 4 378 481 A1**
(43) Date of publication of application: **05.06.2024**
(21) Application number: 22849594.1
(22) Date of filing: 28.07.2022
(51) Int. Cl.: A61K 45/00, A61P 35/00, A61P 43/00, C12N 15/113, A61K 31/133, A61K 31/365, A61K 31/407, A61K 31/58, A61K 31/7048, A61K 31/713

(54) **PHARMACEUTICAL COMPOSITION FOR TREATMENT OF PANCREATIC CANCER AND BILE DUCT CANCER**

(30) Priority: 30.07.2021 JP 2021126154
(71) Applicant: Japan Science and Technology Agency, Kawaguchi-shi, Saitama 332-0012 (JP)
(72) Inventor: FUKAMI Kiyoko, Hachioji-shi, Tokyo 192-0392 (JP); SATOW Reiko, Hachioji-shi, Tokyo 192-0392 (JP)
(74) Representative: Vossius & Partner Patentanwälte Rechtsanwälte mbB
(86) International application number: PCT/JP2022/029163
(87) International publication number: WO 2023/008533

(57) **Abstract**

The present invention provides a pharmaceutical composition that suppresses cell proliferation and has an anti-cancer effect with respect to pancreatic cancer and bile duct cancer. The present invention relates to a pharmaceutical composition containing, as an active ingredient, a substance that reduces an intracellular content of a ZIC5 protein, where the composition is used for treatment of pancreatic cancer or bile duct cancer; the pharmaceutical composition further containing an anti-cancer agent other than the substance that reduces the intracellular content of the ZIC5 protein; and the pharmaceutical composition further containing an anti-cancer agent other than the substance that reduces the intracellular content of the ZIC5 protein.

## Description

### [Technical Field]

The present invention relates to a pharmaceutical composition that suppresses cell proliferation and has an anti-cancer effect with respect to pancreatic cancer and bile duct cancer.

Priority is claimed on Japanese Patent Application No. 2021-126154, filed July 30, 2021, the content of which is incorporated herein by reference.

### [Background Art]

A platelet-derived growth factor (PDGF) is a potent mitogen involved in the development of various cancers, and it is composed of a family consisting of five kinds of disulfide dimers (PDGF-AA, PDGF-AB, PDGF-BB, PDGF-CC, and PDGF-DD). For example, in melanoma, prostate cancer, and colon cancer, cell proliferation is controlled mainly by signal transduction mediated by PDGFD (PDGF-DD).

For example, in melanoma, prostate cancer, and colon cancer, it has been revealed that a ZIC5 (ZIC family member 5 (odd-paired homolog, Drosophila)) protein promotes the expression of PDGFD, which causes activation of focal adhesion kinase (FAK) and signal transducer and activator of transcription 3 (STAT3) and enhances the drug resistance of the melanoma cells, that in a case where the expression of the ZIC5 gene is suppressed, the expression of the PDGFD gene is suppressed, and the cell proliferation of melanoma cells is suppressed, and that ZIC5 also contributes to the enhancement of drug resistance (Non-Patent Document 1 and Non-Patent Document 2). Further, in melanoma, it has also been reported that the ZIC5 protein functions as a transcription factor for E-cadherin, and in a case where the expression of the ZIC5 gene is suppressed, a decrease in the expression level of E-cadherin is suppressed, whereby it is possible to suppress the acquisition of malignancy or metastatic performance (Patent Document 1). It is noted that according to the database of the cancer genome atlas (TCGA) (https://www.cancer.gov/about-nci/organization/ccg/research/structural-genomics/tcga), the expression of ZIC5 is hardly detected in normal tissues other than the brain and the testis in humans, whereas the ZIC5 is highly expressed in various cancer tissues and is also highly expressed in pancreatic cancer and bile duct cancer.

On the other hand, PDGF-BB is known to be involved in pancreatic cancer and bile duct cancer. For example, it has been revealed that in pancreatic cancer cells, a PDGF-BB treatment significantly promotes cell proliferation through a Hippo/YAP signal transduction pathway and also enhances invasiveness (Non-Patent Document 3). In addition, it has been revealed that in bile duct cancer cells, a PDGF-BB treatment promotes a hedgehog (Hh) survival signal, and as a result, the cells are protected from the tumor necrosis factor-related apoptosis-induced ligand (TRAIL)-induced apoptosis (Non-Patent Document 4).

Since both pancreatic cancer and bile duct cancer are present at deep positions in the abdomen, early detection thereof is difficult. They are so-called refractory cancer, and the 5-year survival rate in Japan is as low as about 10% in a case of pancreatic cancer and as low as about 20% even in a case of bile duct cancer. In many cases, these cancers are difficult to be surgically cured since they are already at a metastatic stage at the time of diagnosis, and currently, chemotherapy is mainly selected therefor. In chemotherapy, anti-cancer agents such as gemcitabine, a tegafur/gimeracil/oteracil potassium combination drug, and nab-paclitaxel have been used for pancreatic cancer; however, the fact that resistance to chemotherapy is easily exhibited causes a problem in treatment (Non-Patent Document 5). In addition, in the treatment of bile duct cancer, gemcitabine, cisplatin, and the like have been used in combination as chemotherapy; however, this also has low sensitivity to chemotherapy. A molecule targeted drug, which exhibits high efficacy in both cancers, has not yet been put into practical use.

Pancreatic cancer and bile duct cancer are often associated with inflammations such as pancreatitis and cholangitis, and interleukin-6 (IL-6) or interleukin-22 (IL-22), which are inflammatory cytokines, have been suggested to promote the progression of these cancers (Non-Patent Documents 6 to 9). IL-6 and IL-22 activate signal transducer and activator of transcription 3 (STAT3) in cancer cells. It has been shown that in pancreatic cancer, the STAT3 signal is activated by IL-22, which causes an increase in the expression of genes involved in stem cell properties or transcription factors involved in epithelial-mesenchymal transition (EMT) and enhances the stem cell properties and the tumor forming ability (Non-Patent Document 6). In addition, the expression of STAT3 promotes the metastasis of intrahepatic bile duct cancer and shows a correlation with poor prognosis. It has been revealed that both in vitro and in vivo, the overexpression of STAT3 promotes the invasion, metastasis, and proliferation of the intrahepatic bile duct cancer, and the phosphorylation of STAT3 (Non-Patent Document 9).

### [Citation List]

### [Patent Document]

[Patent Document 1] PCT International Publication No. WO2016/178374

### [Non-Patent Documents]

Non-Patent Document 1: Satow et al., Cancer Research, 2017, vol.77(2), p.366-377.
Non-Patent Document 2: Satow et al., Cancer Science, 2017, vol.108, p.2405-2412, doi: 10.1111/cas. 13419.
Non-Patent Document 3: Li et al., Oncology Reports, 2021, vol.45, p.83-94.
Non-Patent Document 4: Fingas et al., Hepatology, 2011, vol.54(6), p.2076-2088.
Non-Patent Document 5: Kato, Diagnostics (Basel), 2021, vol.11. p.252
Non-Patent Document 6: He et al., Cancer Research, 2018, vol.78(12), p.3293-3305, doi: 10.1158/0008-5472.CAN-17-3131.
Non-Patent Document 7: Fukuda et al., Cancer Cell, 2011, vol.19, p.441-455.
Non-Patent Document 8: Isomoto et al., Gastroenterology, 2007, vol.132, p.384-396.
Non-Patent Document 9: Yang et al., Oncotarget, 2017, vol.8(5), p.7710-7721.

### [Summary of Invention]

### [Technical Problem]

The main object of the present invention is to provide a pharmaceutical composition that suppresses cell proliferation and has an anti-cancer effect with respect to pancreatic cancer and bile duct cancer.

### [Solution to Problem]

As a result of diligent studies, the inventors of the present invention found that in pancreatic cancer and bile duct cancer, in a case of suppressing the intracellular abundance of a ZIC5 protein, cell proliferation is suppressed, and an anti-cancer effect is exhibited, thereby completing the present invention.

That is, a pharmaceutical composition and a ZIC5 protein reducing agent, according to the present invention, are as follows.
[1] A pharmaceutical composition containing, as an active ingredient:
   a substance that reduces an intracellular content of a ZIC5 protein,
   in which the composition is used for treatment of pancreatic cancer or bile duct cancer.
[2] The pharmaceutical composition according to [1],
   in which the substance that reduces the intracellular content of the ZIC5 protein is a substance that suppresses expression of a ZIC5 gene.
[3] The pharmaceutical composition according to [2],
   in which the substance that suppresses the expression of the ZIC5 gene is a nucleic acid molecule that suppresses the expression of the ZIC5 gene by RNA interference.
[4] The pharmaceutical composition according to [1],
   in which the substance that reduces the intracellular content of the ZIC5 protein is any compound represented by General Formula (1) or a derivative thereof, a salt thereof, or a solvate thereof. 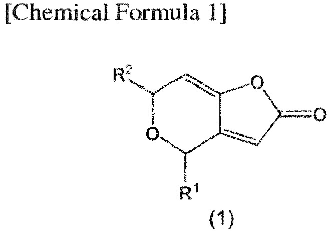 [In the formula, R¹ and R² are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[5] The pharmaceutical composition according to [1],
   in which the substance that reduces the intracellular content of the ZIC5 protein is any compound represented by General Formula (2) or a derivative thereof, a salt thereof, or a solvate thereof. 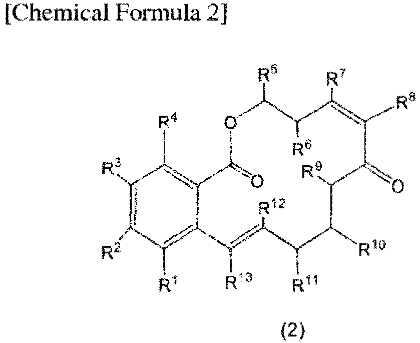 [In the formula, R¹ to R¹³ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[6] The pharmaceutical composition according to [1],
   in which the substance that reduces the intracellular content of the ZIC5 protein is any compound represented by General Formula (3) or a derivative thereof, a salt thereof, or a solvate thereof. 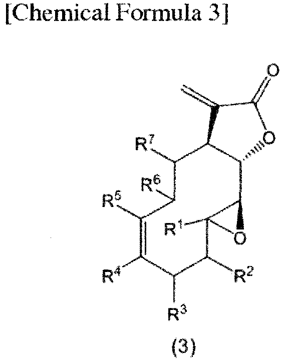 [In the formula, R¹ to R⁷ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[7] The pharmaceutical composition according to [1],
   in which the substance that reduces the intracellular content of the ZIC5 protein is any compound represented by General Formula (4) or a derivative thereof, a salt thereof, or a solvate thereof. 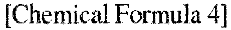 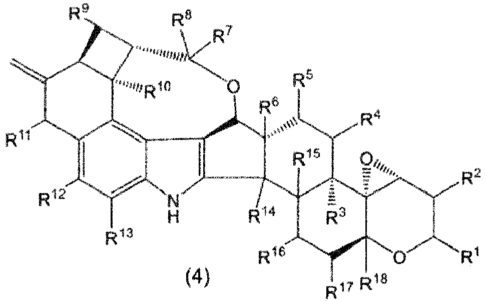 [In the formula, R¹ to R¹⁸ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[8] The pharmaceutical composition according to [1],
   in which the substance that reduces the intracellular content of the ZIC5 protein is any compound represented by General Formula (5) or a derivative thereof, a salt thereof, or a solvate thereof. 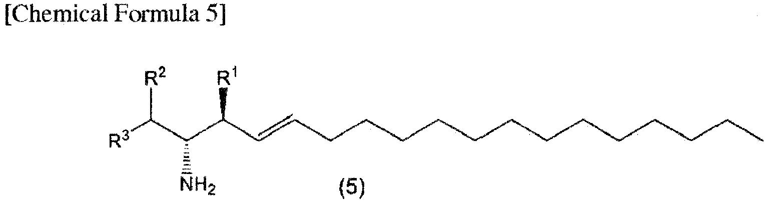 [In the formula, R¹ to R³ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[9] The pharmaceutical composition according to [1],
   in which the substance that reduces the intracellular content of the ZIC5 protein is any compound represented by General Formula (6) or a derivative thereof, a salt thereof, or a solvate thereof. 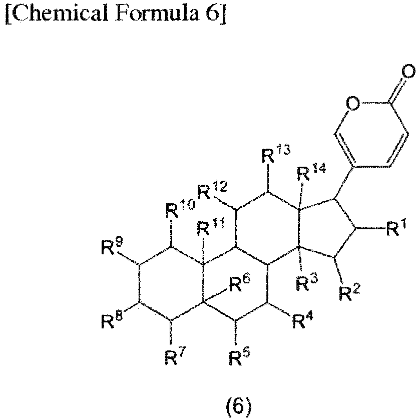 [In the formula, R¹ to R¹⁴ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[10] The pharmaceutical composition according to [1],
   in which the substance that reduces the intracellular content of the ZIC5 protein is any compound represented by General Formula (7) or a derivative thereof, a salt thereof, or a solvate thereof. 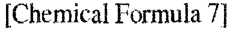 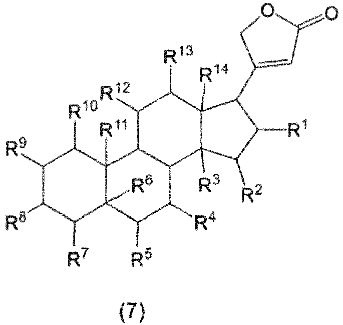 [In the formula, R¹ to R¹⁴ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[11] The pharmaceutical composition according to [1],
   in which the substance that reduces the intracellular content of the ZIC5 protein is any compound represented by General Formula (8) or a derivative thereof, a salt thereof, or a solvate thereof. 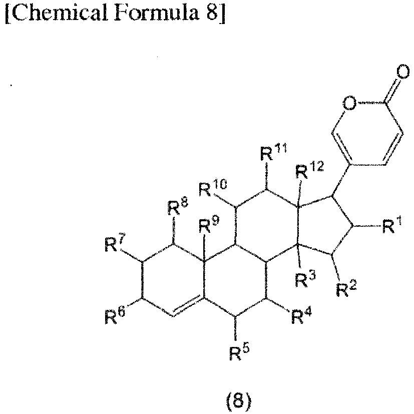 [In the formula, R¹ to R¹² are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[12] The pharmaceutical composition according to [1],
   in which the substance that reduces the intracellular content of the ZIC5 protein is any compound represented by General Formula (9) or a derivative thereof, a salt thereof, or a solvate thereof. 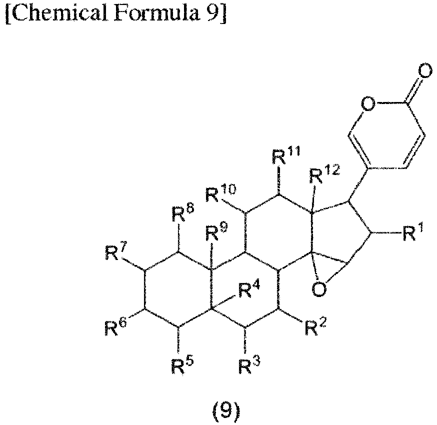 [In the formula, R¹ to R¹² are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.]
[13] The pharmaceutical composition according to any one of [1] to [12],
   in which the pancreatic cancer or the bile duct cancer is a cancer in which the ZIC5 protein is expressed.
[14] The pharmaceutical composition according to any one of [1] to [13], further containing:
   an anti-cancer agent other than the substance that reduces the intracellular content of the ZIC5 protein.
[15] The pharmaceutical composition according to [14],
   in which the anti-cancer agent is one or more anti-cancer agents selected from the group consisting of a BRAF inhibitor and a DNA synthesis inhibitor.
[16] A ZIC5 protein reducing agent including, as an active ingredient:
   a compound represented by any of General Formulae (1) to (9) or a derivative thereof, a salt thereof, or a solvate thereof.

### [Advantageous Effects of Invention]

A pharmaceutical composition and a ZIC5 protein reducing agent, according to the present invention, suppress cell proliferation with respect to pancreatic cancer cells and bile duct cancer cells and have an anti-cancer effect. Therefore, the pharmaceutical composition or the like is a novel agent highly effective for the treatment of pancreatic cancer and bile duct cancer.

### [Brief Description of Drawings]

FIG. 1 is a view showing results obtained by analyzing expression levels of the ZIC5 mRNA in cancer sections and non-cancer sections (not tumor) in clinical specimens of pancreatic cancer (PAAD) and bile duct cancer (CHOL) in Reference Example 1.
FIG. 2 is a view showing results obtained by classifying pancreatic cancer cases and bile duct cancer cases according to the presence or absence of the expression of the ZIC5 gene and carrying out survival analysis in Reference Example 1.
FIG. 3 is a view showing results obtained by analyzing expression levels of the ZIC5 mRNA in human normal tissues, pancreatic cancer cell lines, and bile duct cancer cell lines by real-time PCR in Reference Example 2.
FIG. 4 is a view showing results obtained by investigating an effect on apoptosis induction in a case where the expression of the ZIC5 gene is suppressed in pancreatic cancer cell lines and bile duct cancer cell lines in Example 1.
FIG. 5 is a view showing staining images obtained by subjecting cells to Giemsa staining in Example 1, where the expression of the ZIC5 gene has been suppressed in pancreatic cancer cell lines and bile duct cancer cell lines, gemcitabine (gem) has been subsequently added there to, and culturing has been carried out for 14 days.
FIG. 6 is a view showing results obtained by measuring the relative cell number of cells, where a PANC-1 line (left) and an RBE line (right) have been subjected to transfection with siNeg #1 or siZIC5 #1, subjected to the addition of IL-6 and gemcitabine 2 days later, and then subjected to culturing in Example 2.
FIG. 7 shows Western blotting images of cells of a PANC-1 line and an RBE line, which have been transfected with siNeg #1 or siZIC5 #1 and cultured in the presence of IL-22 or IL-6 in Example 2.
FIG. 8 shows Western blotting images of cells of a PANC-1 line, a MiaPaca-2 line, and an RBE line, which have been transfected with siNeg #1 or siZIC5 #1 in Example 2.
FIG. 9 is a view showing results obtained by quantifying a ZIC5 protein and a GAPDH protein in A375 cells, which have been treated with a compound (T-1) (left) and a compound (T-2) (right) by Western blotting, and measuring a relative amount of the ZIC5 protein ([amount of ZIC5 protein]/[amount of GAPDH protein]) in Example 3,
FIG. 10 is a view showing results obtained by measuring an apoptosis induction rate (%) of each kind of cell, which has been treated with each of the compound (T-1) (upper part) and the compound (T-2) (lower part), in Example 4.
FIG. 11 is a view showing results obtained by measuring an apoptosis induction rate (%) of each kind of cell, which has been treated with each of the compound (T-1) and the compound (T-2), in Example 4.
FIG. 12 is a view showing results obtained by measuring the relative number of cells, which have been treated with the compound (T-1) (left) and the compound (T-2) (right) and then treated with a BRAF inhibitor (Vem), in Example 5.
FIG. 13 is a view showing results obtained by measuring the relative cell number of cells transfected with a ZIC5 expression plasmid (Z5) to overexpress a ZIC5 protein with respect to cells treated with the compound (T-1) (left) and the compound (T-2) (right), the cells having been transfected with an empty vector (C) in Example 6.
FIG. 14 is a view showing results obtained by measuring a proliferation rate of pancreatic cancer cells in Example 7, in a case where the cells have been subjected to viral introduction with shZIC5/pSIREN-GFP or shNeg/pSIREN-GFP and has been cultured for 3 days.
FIG. 15 is a view showing results obtained by measuring an expression level of the ZIC5 mRNA in each kind of cell after treatment with dox for 48 hours in Example 8, where the cells are MiaPaca-2 cells into which tet-shZIC5 has been introduced and MiaPaca-2 cells into which tet-shNeg has been introduced.
FIG. 16 is a view showing results obtained by time-dependently measuring volumes of tumors in immunodeficient mice transplanted with MiaPaca-2 cells into which tet-shZIC5 has been introduced and MiaPaca-2 cells into which tet-shNeg has been introduced, in Example 8.

### [Description of Embodiments]

A pharmaceutical composition according to the present invention is characterized in that it contains, as an active ingredient, a substance (hereinafter, may be referred to as a "ZIC5 protein reducing substance") that reduces an intracellular content of a ZIC5 protein and is used for the treatment of pancreatic cancer or bile duct cancer. The ZIC5 protein is a protein belonging to the Zic family and has five C2H2-type zinc finger motifs. In pancreatic cancer cells and bile duct cancer cells, in a case where the intracellular content of the ZIC5 protein is reduced, cell proliferation is suppressed, and an anti-cancer effect is obtained.

The mechanism of action of the ZIC5 protein leading to the suppression of cell proliferation in pancreatic cancer cells and bile duct cancer cells is not clear. However, The ZIC5 protein may function as a transcription factor for proteins that contribute to proliferation signals in pancreatic cancer cells and bile duct cancer cells. It is noted that in melanoma, colon cancer, and prostate cancer, ZIC5 is involved in proliferation signals mediated by PDGFD, and in a case where the expression of the ZIC5 gene is suppressed, the expression of the PDGFD gene is suppressed, and the cell proliferation of melanoma cells is suppressed. On the other hand, in pancreatic cancer and bile duct cancer, not only signal transduction mediated by PDGFD but also signal transduction mainly mediated by PDGF-BB are involved in the proliferation and the survival.

The reduction of the intracellular content of the ZIC5 protein can be achieved, for example, by inhibiting the expression of the ZIC5 gene. That is, examples of the ZIC5 protein reducing substance, which is an active ingredient of the pharmaceutical composition according to the present invention, include a substance having the action of suppressing the expression itself of the ZIC5 gene by RNA interference or the like. Examples of the substance include a nucleic acid that directly inhibits the expression of the ZIC5 gene, such as a small interfering RNA (siRNA), a short hairpin RNA (shRNA), or microRNA (miRNA), which has a double-stranded structure consisting of a sense strand an antisense strand of a partial region (an RNA interference (RNA) target region) of the cDNA of the ZIC5 gene. In addition, an RNAi-inducing vector that is capable of producing siRNA or the like in tumor cells which are targets may be used. Regarding the construction of an siRNA-inducing, shRNA-inducing, miRNA-inducing, or RNAi-inducing vector, it can be designed and produced based on the base sequence information of the cDNA of the ZIC5 gene to be targeted, according to a conventional method. In addition, an RNAi-inducing vector can also be produced by inserting a base sequence of an RNAi target region into a base sequence of various commercially available RNAi vectors.

The ZIC5 protein reducing substance that is used in the present invention may be a substance that degrades the ZIC5 protein. In a case where the ZIC5 protein itself is degraded, the function thereof is inhibited. The substance that degrades the ZIC5 protein may be a degrading enzyme that directly degrades the ZIC5 protein, or it may be a substance that provides various kinds of labels such as polyubiquitin so that the ZIC5 protein becomes a substrate for the proteolytic enzyme. The substance may be a protein or a peptide, may be a nucleic acid, or may be a low molecular weight compound.

The ZIC5 protein reducing substance can be obtained, for example, from a compound library by carrying out screening. The compound library may be a nucleic acid library, may be a peptide library, or may be a low molecular weight compound library. Cells overexpressing the ZIC5 protein are treated with each of compounds constituting a library, and a compound that reduces the amount of the ZIC5 protein in cells can be selected as a candidate compound of the ZIC5 protein reducing substance.

Specific examples of the ZIC5 protein reducing substance, which is a low molecular weight compound, include compounds represented by General Formulae (1) to (9). Hereinafter, each of these compounds may be referred to as a "ZIC5 protein reducing agent".

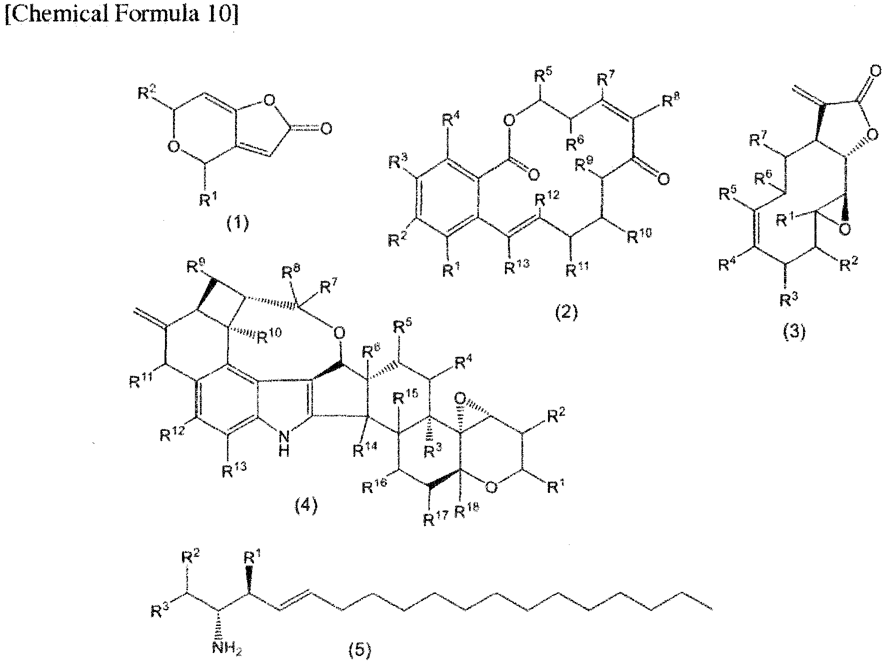

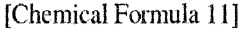

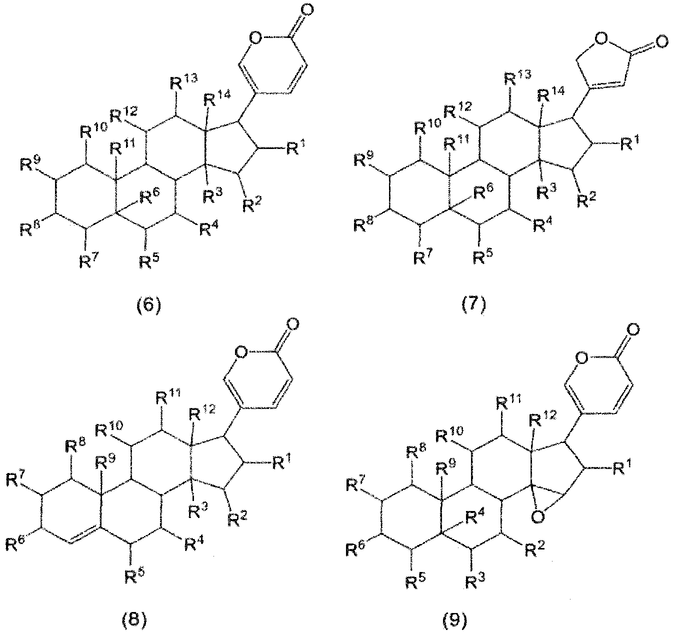

In General Formulae (1) to (9), R¹ to R¹⁸ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent.

Examples of the halogen atom include a fluorine atom, a chlorine atom, a bromine atom, and an iodine atom. In the present invention, the halogen atom as R¹ to R¹⁸ is preferably a chlorine atom.

The alkyl group having 1 to 6 carbon atoms may be linear, may be branched, or may be a group having a cyclic structure; however, it is preferably a linear or branched group. Specific examples thereof include a methyl group, an ethyl group, a propyl group, an isopropyl group, an n-butyl group, an isobutyl group, a t-butyl group, a pentyl group, an isoamyl group, a hexyl group, and a cyclohexyl group. In the present invention, the alkyl group as R¹ to R¹⁸ is preferably an alkyl group having 1 to 3 carbon atoms, and more preferably a linear alkyl group having 1 to 3 carbon atoms.

The alkenyl group having 2 to 6 carbon atoms may be linear, may be branched, or may be a group having a cyclic structure; however, it is preferably a linear or branched group. Specific examples thereof include an ethenyl group (a vinyl group), a 1-propenyl group, a 2-propenyl group (an allyl group), a 1-butenyl group, a 2-butenyl group, a 3-butenyl group, a pentenyl group, and a hexenyl group. In the present invention, the alkenyl group as R¹ to R¹⁸ is preferably an alkenyl group having 2 to 4 carbon atoms, and more preferably an alkenyl group having 2 or 3 carbon atoms.

The hydroxyalkyl group is preferably a group obtained by substituting at least one hydrogen atom among hydrogen atoms bonded to a carbon atom of an alkyl group having 1 to 6 carbon atoms with a hydroxy group, more preferably a group obtained by substituting one of hydrogen atoms bonded to a carbon atom of an alkyl group having 1 to 6 carbon atoms with a hydroxy group, and still more preferably a group obtained by substituting one of hydrogen atoms bonded to a carbon atom of an alkyl group having 1 to 3 carbon atoms with a hydroxy group. Specific examples thereof include a hydroxymethyl group, a 1-hydroxyethyl group, a 2-hydroxyethyl group, a 1-hydroxypropyl group, a 2-hydroxypropyl group, a 3-hydroxypropyl group, a 1-hydroxybutyl group, a 2-hydroxybutyl group, a 3-hydroxybutyl group, and 4-hydroxybutyl group.

The alkoxy group is preferably a group in which the alkyl group moiety is an alkyl group having 1 to 6 carbon atoms, more preferably a group in which the alkyl group moiety is an alkyl group having 1 to 3 carbon atoms, and still more preferably a group in which the alkyl group moiety is a linear alkyl group having 1 to 3 carbon atoms. Specific examples thereof include a methoxy group, an ethoxy group, an n-propoxy group, an isopropoxy group, an n-butoxy group, an isobutoxy group, a t-butoxy group, an s-butoxy group, an n-pentyloxy group, and an n-hexyloxy group.

The acyloxy group is preferably an acyloxy group having 2 to 7 carbon atoms, and more preferably an acyloxy group having 2 to 4 carbon atoms. Specific examples thereof include an acetyloxy group, a propanoyloxy group, an n-butanoyloxy group, a 2-methylpropanoyloxy group, an n-pentanoyloxy group, a 2,2-dimethylpropanoyloxy group, and n-hexanoyl oxy group.

Examples of the tetrahydropyranyloxy group having a substituent include a group obtained by substituting 1 to 4 hydrogen atoms among hydrogen atoms bonded to a carbon atom of a tetrahydropyranyloxy group, with a halogen atom, a hydroxy group, an alkyl group having 1 to 6 carbon atoms, an alkenyl group having 2 to 6 carbon atoms, a hydroxyalkyl group, a formyl group, an acyloxy group, or a tetrahydropyranyloxy group which may have a substituent. As these substituents, the same groups as those exemplified above can be used. In a case where two or more hydrogen atoms are substituted, the substituents may be groups of the same kind or may be groups of kinds different from each other. In the present invention, a group, in which among hydrogen atoms bonded to a carbon atom of a tetrahydropyranyloxy group, 1 to 4 hydrogen atoms are substituted with a hydroxy group, an alkyl group having 1 to 6 carbon atoms, a hydroxyalkyl group having 1 to 6 carbon atoms, or a tetrahydropyranyloxy group having a substituent, is preferable, and a group, in which among hydrogen atoms bonded to a carbon atom of a tetrahydropyranyloxy group, 1 to 4 hydrogen atoms are substituted with a group in which among hydrogen atoms bonded to a carbon atom of a hydroxy group, a methyl group, an ethyl group, a propyl group, an isopropyl group, a hydroxymethyl group, a hydroxyethyl group, a hydroxypropyl group, a hydroxyisopropyl group, or a tetrahydropyranyloxy group, 1 to 4 hydrogen atoms are substituted with a hydroxy group, a methyl group, or a hydroxymethyl group, is more preferable. Among the above, it is preferably a group in which among hydrogen atoms bonded to a carbon atom of a tetrahydropyranyloxy group, three hydrogen atoms are substituted with a hydroxy group, and one hydrogen atom is substituted with a methyl group, an ethyl group, a propyl group, an isopropyl group, a hydroxymethyl group, a hydroxyethyl group, a hydroxypropyl group, or a hydroxyisopropyl group, or a group in which among hydrogen atoms bonded to a carbon atom of a tetrahydropyranyloxy group, two hydrogen atoms are substituted with a hydroxy group, one hydrogen atom is substituted with a methyl group, an ethyl group, a propyl group, an isopropyl group, a hydroxymethyl group, a hydroxyethyl group, or a hydroxypropyl group, and one hydrogen atom is substituted with a group in which among hydrogen atoms bonded to a carbon atom of a tetrahydropyranyloxy group, 1 to 4 hydrogen atoms are substituted with a hydroxy group, a methyl group, or a hydroxymethyl group.

The compound represented by General Formula (1) is preferably a compound in which R¹ and R² are each independently a hydrogen atom, a hydroxy group, or an alkyl group having 1 to 3 carbon atoms, and it is more preferably a compound in which R¹ is a hydroxy group and R² is a hydrogen atom or an alkyl group having 1 to 3 carbon atoms, or a compound in which R¹ is a hydrogen atom or an alkyl group having 1 to 3 carbon atoms and R² is a hydroxy group.

The compound represented by General Formula (2) is preferably a compound in which R¹ to R¹³ are each independently a hydrogen atom, a hydroxy group, an alkyl group having 1 to 3 carbon atoms, or an alkoxy group having 1 to 3 carbon atoms, more preferably a compound in which at least one of R¹ to R¹³ is a hydroxy group, and R¹ to R⁴ are each independently a hydrogen atom, a hydroxy group, or an alkoxy group having 1 to 3 carbon atoms, and R⁵ to R¹³ are each independently a hydrogen atom or an alkyl group having 1 to 3 carbon atoms, and still more preferably a compound in which R¹ to R³ are each independently a hydrogen atom or an alkoxy group having 1 to 3 carbon atoms, R⁴, R⁹, and R¹⁰ are a hydroxy group, and R⁵ to R⁸ and R¹¹ to R¹³ are each independently a hydrogen atom or an alkyl group having 1 to 3 carbon atoms.

The compound represented by General Formula (3) is preferably a compound in which R¹ to R⁷ are each independently a hydrogen atom, a hydroxy group, or an alkyl group having 1 to 3 carbon atoms, and it is more preferably a compound in which R¹ to R⁷ are each independently a hydrogen atom or an alkyl group having 1 to 3 carbon atoms,

The compound represented by General Formula (4) is preferably a compound in which at least one of R¹ to R¹⁸ is a hydroxy group, and R¹ to R¹⁸ are each independently a hydrogen atom, a halogen atom, a hydroxy group, an alkyl group having 1 to 3 carbon atoms, or an alkenyl group having 2 or 3 carbon atoms, and it is more preferably a compound in which R² and R⁹ are a hydroxy group, and R¹, R³ to R⁸, and R¹⁰ to R¹⁸ are each independently a hydrogen atom, a halogen atom, an alkyl group having 1 or 3 carbon atoms, or an alkenyl group having 2 or 3 carbon atoms.

The compound represented by General Formula (5) is preferably a compound in which at least one of R¹ to R³ is a hydroxy group, more preferably a compound in which at least one of R¹ to R³ is a hydroxy group, and R¹ to R³ are each independently a hydrogen atom, a hydroxy group, or an alkyl group having 1 to 3 carbon atoms, and still more preferably a compound in which R¹ and R³ are a hydroxy group, and R² is a hydrogen atom or an alkyl group having 1 to 3 carbon atoms.

The compound represented by General Formula (6) is preferably a compound in which at least one of R¹ to R¹⁴ is a hydroxy group, more preferably a compound in which at least one of R¹ to R¹⁴ is a hydroxy group, and R¹ to R¹⁴ are each independently a hydrogen atom, a hydroxy group, an alkyl group having 1 to 3 carbon atoms, a hydroxyalkyl group having 1 to 3 carbon atoms, a formyl group, an acyloxy group having 1 to 4 carbon atoms, or a tetrahydropyranyloxy group which may have a substituent, and still more preferably a compound in which R³ and R⁶ are a hydroxy group, R⁸ is a tetrahydropyranyloxy group which may have a substituent, and R¹, R², R⁴ to R⁷, and R⁹ to R¹⁴ are each independently a hydrogen atom, an alkyl group having 1 to 3 carbon atoms, a hydroxyalkyl group having 1 to 3 carbon atoms, a formyl group, or an acyloxy group having 1 to 4 carbon atoms, or a compound in which R³ and R⁸ are a hydroxy group, and R¹, R², R⁴ to R⁷, and R⁹ to R¹⁴ are each independently a hydrogen atom, an alkyl group having 1 to 3 carbon atoms, a hydroxyalkyl group having 1 to 3 carbon atoms, a formyl group, or an acyloxy group having 1 to 4 carbon atoms.

The compound represented by General Formula (7) is preferably a compound in which at least one of R¹ to R¹⁴ is a hydroxy group, more preferably a compound in which at least one of R¹ to R¹⁴ is a hydroxy group, and R¹ to R¹⁴ are each independently a hydrogen atom, a hydroxy group, an alkyl group having 1 to 3 carbon atoms, a hydroxyalkyl group having 1 to 3 carbon atoms, a formyl group, an acyloxy group having 1 to 4 carbon atoms, or a tetrahydropyranyloxy group which may have a substituent, and still more preferably a compound in which R³, R⁶, R¹⁰, and R¹² are a hydroxy group, R⁸ is a tetrahydropyranyloxy group which may have a substituent, and R¹, R², R⁴, R⁵, R⁷, R⁹, R¹¹, R¹³, and R¹⁴ are each independently a hydrogen atom, an alkyl group having 1 to 3 carbon atoms, a hydroxyalkyl group having 1 to 3 carbon atoms, a formyl group, or an acyloxy group having 1 to 4 carbon atoms.

The compound represented by General Formula (8) is preferably a compound in which at least one of R¹ to R¹² is a hydroxy group, more preferably a compound in which at least one of R¹ to R¹² is a hydroxy group, and R¹ to R¹² are each independently a hydrogen atom, a hydroxy group, an alkyl group having 1 to 3 carbon atoms, a hydroxyalkyl group having 1 to 3 carbon atoms, a formyl group, an acyloxy group having 1 to 4 carbon atoms, or a tetrahydropyranyloxy group which may have a substituent, and still more preferably a compound in which R³ is a hydroxy group, R⁶ is a tetrahydropyranyloxy group which may have a substituent, and R¹, R², R⁴, R⁵, and R⁷ to R¹² are each independently a hydrogen atom, an alkyl group having 1 to 3 carbon atoms, a hydroxyalkyl group having 1 to 3 carbon atoms, a formyl group, or an acyloxy group having 1 to 4 carbon atoms.

The compound represented by General Formula (9) is preferably a compound in which at least one of R¹ to R¹² is a hydroxy group, more preferably a compound in which at least one of R¹ to R¹² is a hydroxy group, and R¹ to R¹² are each independently a hydrogen atom, a hydroxy group, an alkyl group having 1 to 3 carbon atoms, a hydroxyalkyl group having 1 to 3 carbon atoms, a formyl group, an acyloxy group having 1 to 4 carbon atoms, or a tetrahydropyranyloxy group which may have a substituent, and still more preferably a compound in which R⁶ is a hydroxy group, R⁴ is a hydroxy group or a hydrogen atom, and R¹ to R³, R⁵, and R⁷ to R¹² are each independently a hydrogen atom, an alkyl group having 1 to 3 carbon atoms, a hydroxyalkyl group having 1 to 3 carbon atoms, a formyl group, or an acyloxy group having 1 to 4 carbon atoms.

As the ZIC5 protein reducing substance that is used in the present invention, the compound represented by any of General Formulae (1) to (9) may form a salt. Examples of the acid or base that forms the salt include mineral acids such as hydrochloric acid and sulfuric acid; organic acids such as acetic acid, succinic acid, and citric acid; alkali metals such as sodium and potassium; and alkaline earth metals such as calcium and magnesium. In addition, the compound represented by any of General Formulae (1) to (9) may be in a form of a solvate such as a hydrate.

The low molecular weight compound among the ZIC5 protein reducing substances that are used in the present invention may be a derivative of the compound represented by any of General Formulae (1) to (9). Examples of the derivative include compounds in which any group of R¹ to R¹⁸ in General Formulae (1) to (9) has been subjected to modification with various functional groups, oxidation, reduction, or atom substitution. The ZIC5 protein reducing substance is more preferably a derivative that is obtained by subjecting the compound represented by any of General Formulae (1) to (9) to prodruging, which is carried out for pharmaceutical drug. Examples of the derivative include a monophosphoramidite derivative in which the hydroxy group among R¹ to R¹⁸ in General Formulae (1) to (9) is substituted with a monophosphoramidite group, and a monophosphorothioate derivative in which the hydroxy group is substituted with a monophosphorothioate group. In addition, as the ZIC5 protein reducing substance that is used in the present invention, the derivative of the compound represented by any of General Formulae (1) to (9) may form a salt and may be in a form of a solvate such as a hydrate. As the salt, those listed above can be used.

Even in a case of using a substance that suppresses or inhibits the function of the ZIC5 protein by directly or indirectly binding to the ZIC5 protein, instead of the ZIC5 protein reducing substance, the cell proliferation of pancreatic cancer cells and bile duct cancer cells is suppressed, and the anti-cancer effect is obtained. The substance is not particularly limited and may be any of a nucleic acid, a peptide, a protein, or a low molecular weight compound.

Examples of the substance that suppresses or inhibits the function of the ZIC5 protein by directly or indirectly binding to the ZIC5 protein include a nucleic acid that binds to the ZIC5 protein to inhibit the interaction between the ZIC5 protein and a promoter sequence of a target gene, for which the ZIC5 protein functions as a transcription factor. Examples of the nucleic acid that binds to the ZIC5 protein to inhibit the interaction between the ZIC5 protein and a promoter sequence of a target gene, for which the ZIC5 protein functions as a transcription factor, include a nucleic acid molecule (so-called decoy nucleic acid) that has a base sequence that is identical or highly homologous to a ZIC5 protein binding region in a promoter sequence of a target gene. The decoy nucleic acid may be DNA or may be RNA, and it may be a single-stranded nucleic acid or may be a double-stranded nucleic acid. The decoy nucleic acid is preferably a double-stranded DNA since it is excellent in stability in vivo.

Examples of the protein that binds to the ZIC5 protein to inhibit the intracellular function of the ZIC5 protein include an anti-ZIC5 antibody. The antibody may be a monoclonal antibody or may be a polyclonal antibody. In addition, it may be an artificially synthesized antibody such as a chimeric antibody, a single chain antibody, or a humanized antibody. These antibodies can be produced according to conventional methods.

Even in a case of using a substance that inhibits the nuclear localization of the ZIC5 protein, instead of the ZIC5 protein reducing substance, the cell proliferation of pancreatic cancer cells and bile duct cancer cells is suppressed, and the anti-cancer effect is obtained. In a case where the nuclear localization of the ZIC5 protein is inhibited, the function of the ZIC5 protein as a transcription factor is inhibited. The substance that inhibits the nuclear localization of the ZIC5 protein may be a substance that inhibits the nuclear translocation of the ZIC5 protein or may be a substance that promotes the discharge of the ZIC5 protein to the outside of the nucleus. Examples of the substance that inhibits the nuclear translocation include a substance that binds to the ZIC5 protein so that the nuclear translocation signal of the ZIC5 protein is covered. The substance may be a protein or a peptide or may be a low molecular weight compound.

The pharmaceutical composition according to the present invention can be formulated into formulation forms suitable for various administration forms, such as oral administration, intravenous injection, direct nasal or oral administration, and transdermal administration, according to conventional methods. Examples of the formulation forms include a tablet, a powder, a granule, a capsule, a chewable preparation, a syrup, a liquid preparation, a suspension, an injection agent, a gargling agent, a spray, a patch, and an ointment.

The pharmaceutical composition according to the present invention may contain various additives in addition to the ZIC5 protein reducing substance, which is an active ingredient. Examples of the additives include an excipient, a binding agent, a lubricant, a wetting agent, a solvent, a disintegrating agent, a dissolution assisting agent, a suspending agent, an emulsifying agent, an isotonizing agent, a stabilizer, a buffering agent, a preservative, an antioxidant, a flavoring agent, and a coloring agent. For these additives, additives can be used by being appropriately selected from those which are pharmaceutically acceptable substances and are used in the formulation of medicinal drugs, and are used in the formulation of medicinal drugs.

The pharmaceutical composition according to the present invention may contain another anti-cancer agent in addition to the ZIC5 protein reducing substance, which is an active ingredient. A higher treatment effect can be easily obtained in a case of using an anti-cancer agent in combination, which exhibits anti-cancer action by a mechanism of action different from that of the ZIC5 protein reducing substance. The other anti-cancer agent is not particularly limited, and examples thereof include a BRAF inhibitor and a DNA synthesis inhibitor. Examples of the BRAF inhibitor include vemurafenib, dabrafenib, and encorafenib. In addition, examples of the DNA synthesis inhibitor include pyrimidine analogs such as gemcitabine and cytarabine; purine analogs such as fludarabine and cladribine: dihydrofolate reductase inhibitors such as methotrexate; thymidylate synthase inhibitors such as 5-fluorouracil, tegafur, and capecitabine; IMP dehydrogenase inhibitors such as 6-mercaptopurine; adenosine deaminase inhibitors such as pentostatin; ribonucleotide reductase inhibitors such as hydroxycarbamide; and methotrexate antidotes such as calcium folinate. The other anti-cancer agent contained in the pharmaceutical composition of the present invention may be one kind or may be two or more kinds.

In a case of administering the pharmaceutical composition according to the present invention to a human or an animal other than the human and reducing the amount of the ZIC5 protein in pancreatic cancer cells or bile duct cancer cells, it is possible to suppress cell proliferation with respect to pancreatic cancer cells and bile duct cancer cells and obtain an anti-cancer effect. The cancer to be treated by the pharmaceutical composition according to the present invention is not particularly limited as long as it is pancreatic cancer or bile duct cancer; however, the cancer is preferably a cancer in which the ZIC5 protein is expressed. The pancreatic cancer or the bile duct cancer, in which the ZIC5 protein is expressed, may be primary cancer or may be metastatic cancer.

Even in pancreatic cancer cells or bile duct cancer cells having a high content of inflammatory cytokines such as IL-6, an effect of suppressing cell proliferation can be obtained by the reduction of the amount of the ZIC5 protein in cells. Therefore, the pancreatic cancer and the bile duct cancer, in which the pharmaceutical composition according to the present invention is administered to obtain an anti-cancer effect, may be a cancer in which inflammation has occurred.

The animal to which the pharmaceutical composition according to the present invention is administered is not particularly limited and may be a human being or an animal other than a human being. Examples of the non-human animal include mammals such as a cow, a pig, a horse, a sheep, a goat, a monkey, a dog, a cat, a rabbit, a mouse, a rat, a hamster, and a guinea pig; and birds such as a chicken, a quail, and a duck.

The amount of the ZIC5 protein in cells may be measured at an mRNA level or may be measured at a protein level. The method of measuring the amount of the ZIC5 protein in cells is not particularly limited as long as it is a method that is capable of quantitatively or semi-quantitatively measuring an amount of a target protein or an amount of a target mRNA in cells, and it can be used by being appropriately selected from known methods that are used for detecting mRNA or protein in a specimen. Each method can be carried out according to a conventional method.

The amount of the mRNA of the ZIC5 gene may be detected by a hybridization method that uses a probe that can hybridize with the mRNA of the ZIC5 gene or may be detected by a method that uses a nucleic acid amplification reaction using a primer that can hybridize with the mRNA of the ZIC5 gene and a polymerase. In addition, a commercially available detection kit or the like can also be used. For example, after a reverse transcription reaction is carried out using total RNA extracted from test tumor cells as a template to synthesize cDNA, a polymerase chain reaction (PCR) or the like is carried out using the obtained cDNA as a template, and the amount of the obtained amplification product is measured, whereby the amount of the mRNA of the ZIC5 gene can be quantified. The amount of the amplification product can be quantitatively measured by specifically separating the amplification product by using gel or capillary electrophoresis and then detecting it. In addition, in a case of carrying out semiquantitative PCR such as real-time PCR instead of PCR, the detection of the mRNA of the ZIC5 gene and the quantification thereof can be easily carried out at the same time.

The amount of the ZIC5 protein can be measured by using an antibody (an anti-ZIC5 antibody) that specifically binds to the ZIC5 protein. For example, immunostaining is carried out using an anti-ZIC5 antibody as a primary antibody to investigate the presence or absence of the expression of ZIC5 or the expression intensity thereof in test tumor cells, based on the presence or absence of the staining or staining intensity. The immunostaining may be an enzyme antibody staining method in which an enzyme-labeled antibody is used or may be a fluorescence antibody staining method in which a fluorescently labeled antibody is used. In addition, the amount of the ZIC5 protein in test tumor cells can be quantitatively measured by separating proteins in a cell extract prepared from the test tumor cells by SDS-PAGE or the like and then carrying out a Western blotting method or the like.

### [Examples]

Next, the present invention will be described in more detail by showing Examples; however, the present invention is not limited to Examples below.

### <Cell culture>

In Examples and the like described later, the cells used were cultured as follows.

Regarding three kinds of pancreatic cancer cell lines (an AsPC-1 line, a Panc-1 line, and a MiaPaca-2 line) and one kind of bile duct cancer cell line (an RBE line), those distributed from American Type Culture Collection (ATCC), were used. These cells were cultured in an RPMI 1640 medium (manufactured by Invitrogen Corporation) containing 10% fetal bovine serum, at 37°C in the presence of 5% CO₂.

### <Measurement of apoptosis induction rate>

The apoptosis induction rate (%) was measured as follows. First, a CellEvent Caspase-3/7 Green Detection Reagent (manufactured by Invitrogen Corporation) and Hoechst 33342 were added to each kind of cell, followed by incubation for 40 minutes. Next, the staining state of the cells after incubation was analyzed by using a DAPI filter and an FITC filter of an imaging cytometer "IN Cell Analyzer 2000" (manufactured by GE Healthcare). The proportion (%) of Caspase-3/7 active cells (CellEvent Caspase-3/7 Green Detection Reagent positive cells) with respect to all DAPI-stained cells (DAPI positive cells) was determined by "IN Cell Analyzer Workstation 3.7" (manufactured by GE Healthcare), and this was defined as the apoptosis induction rate (%). The statistical difference was determined according to the Tukey's multiple comparison test (n = 3, *p < 0.05, **p < 0.01, ***p < 0.001).

### [Reference Example 1]

The data on the TCGA database was analyzed to analyze the expression level of the ZIC5 mRNA in clinical specimens of pancreatic cancer and bile duct cancer.

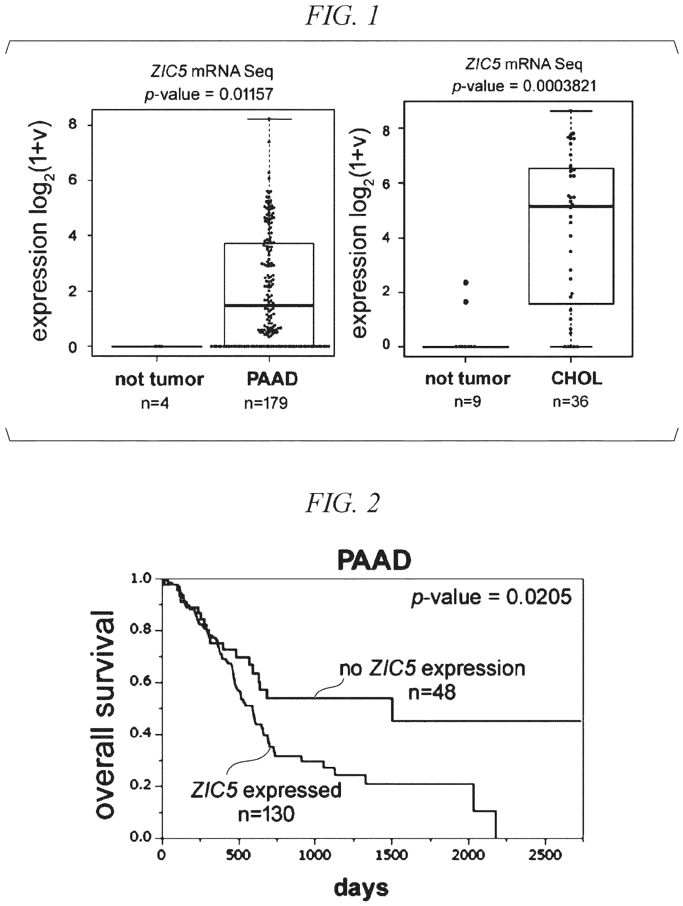

First, RNA sequence data in clinical specimens of pancreatic cancer (PAAD) and bile duct cancer (CHOL) were acquired from the TCGA database. Analysis software JMP was used to subject the collected sequence data to the analysis of expression levels of the ZIC5 gene in non-cancer tissues and cancer tissues. Specifically, the RNA sequence data of the cancer tissues of pancreatic cancer (n = 179) and the non-cancer sections corresponding thereto (not tumor) (n = 4) was subjected to the Mann-Whitney U test. The results are shown in FIG. 1 (left figure). In addition, the RNA sequence data of the cancer tissues of bile duct cancer (n = 36) and the non-cancer sections corresponding thereto (not tumor) (n = 9) was subjected to the same analysis. The results are shown in FIG. 1 (right figure). As a result, it was found that the ZIC5 expression level is clearly higher in the cancer tissues than in the non-cancer tissues.

Subsequently, clinical data was acquired from the TCGA database, and pancreatic cancer (PAAD) cases were classified according to the presence or absence of the expression of the ZIC5 gene to verify whether the expression of the ZIC5 gene is involved in the survival time. The PAAD cases (n = 130) in which the ZIC5 gene was expressed and the non-cancer section cases (not tumor) (n = 48) corresponding thereto were analyzed for the survival analysis. The results are shown in FIG. 2. As a result, it was revealed that in the pancreatic cancer, the survival time is significantly decreased due to the expression of the ZIC5 gene.

### [Reference Example 2]

In the Human Protein Atlas (THPA) database (https://www.proteinatlas.org/humanproteome/tissue), it is suggested that the ZIC5 mRNA is detected only in the cerebral cortex and the testis among the normal adult human tissues but is not detected in other normal tissues; however, no report has verified this by quantitative PCR. Therefore, the ZIC5 expression levels in a human normal tissue-derived cDNA (manufactured by Takara Bio Inc.), three kinds of pancreatic cancer cell lines (an AsPC-1 line, a Panc-1 line, and a MiaPaca-2 line), and one kind of bile duct cancer cell line (an RBE line) were verified by quantitative PCR.

### <Measurement of ZIC5 expression level by qRT-PCR>

The amount of the mRNA of the ZIC5 gene in each kind of the cultured cell line was measured by qRT-PCR. The mRNA level of the glyceraldehyde-3-phosphate dehydrogenase (GAPDH) gene was used as an internal standard. The measurement was carried out by two independent trials.

First, the total RNA of each kind of cell was recovered, and a reverse transcription reaction was carried out using the total RNA as a template to synthesize cDNA. A commercially available kit "ReliaPrep RNA Cell Miniprep System" (manufactured by Promega Corporation) was used for recovering total RNA, and a commercially available kit "High Capacity cDNA Reverse Transcription kit" (manufactured by Applied Biosystems) was used for a reverse transcription reaction, where each of them was used according to the protocol attached to the product.

Next, real-time PCR was carried out using the obtained cDNA and the human normal tissue-derived cDNA as templates. The real-time PCR was carried out using a commercially available kit "THUNDERBIRD SYBR qPCR Mix" (manufactured by TOYOBO Co., Ltd.) and a thermal cycler "CFX96" (manufactured by Bio-Rad Laboratories Inc.). The primers used are shown in Table 1.

**[Table 1]**

| Primer | Base Sequence | Sequence Number |
|---|---|---|
| ZIC5 forward | AACCTCAAGATCCACAAGCGT | 1 |
| ZIC5 reverse | CACTGGTGTGGACATGGGAA | 2 |
| GAPDH forward | AGCCTCCCGCTTCGCTCTCT | 3 |
| GAPDH reverse | CCAGGCGCCCAATACGACCA | 4 |

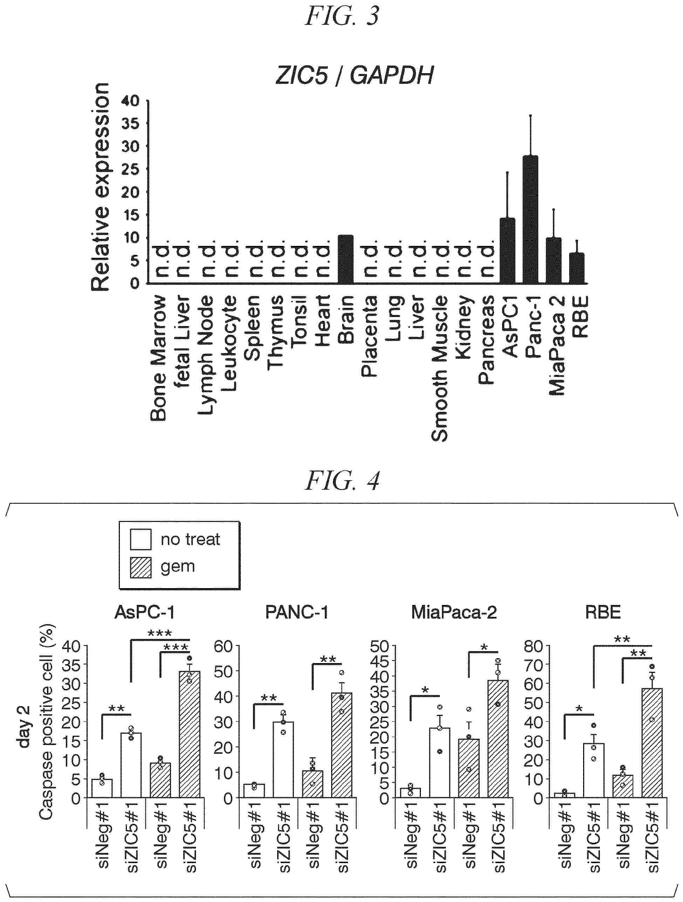

The mRNA levels of the ZIC5 gene in each kind of cell and the human normal tissues were normalized by the mRNA level of the GAPDH gene. FIG. 3 shows the average value (n = 3) of the relative expression level of the ZIC5 gene ([amount of mRNA of ZIC5 gene]/[amount of mRNA of GAPDH gene]). In the figure, "n.d." means "not detected". As a result, among the human normal tissues, the expression thereof was detected only in the brain but was not detected in other tissues (bone marrow, liver, lymph nodes, leukocytes, spleen, thymus, tonsil, heart, placenta, lung, smooth muscle, kidney, and pancreas). The expression of the ZIC5 gene was detected in any of the pancreatic cancer cell lines (the AsPC-1 line, the Panc-1 line, and the MiaPaca-2 line) and the bile duct cancer cell line (the RBE line). From these results, it was shown that the ZIC5 gene has very low expression in many human normal tissues.

### [Example 1]

It was verified whether the ZIC5 protein affects survival in pancreatic cancer and bile duct cancer. Specifically, regarding three kinds of pancreatic cancer cell lines (an AsPC-1 line, a Pane-1 line, and a MiaPaca-2 line) and one kind of bile duct cancer cell line (an RBE line), the expression of the endogenous ZIC5 gene was suppressed by RNA interference, and the proportion of apoptotic cells was investigated.

First, each kind of cell was transfected with two kinds of control siRNAs (siNeg #1 and siNeg #2) and two kinds of siRNAs (siZIC5 #1 and siZIC5 #2) targeting the mRNA of the ZIC5 gene listed in Table 2 and cultured for 72 hours, and then the proportion of cells that had undergone apoptosis (apoptosis induction rate) (%) was investigated. The apoptosis induction rate was detected using a CellEvent Caspase-3/7 Green Detection Reagent (manufactured by Invitrogen Corporation). The results are shown in FIG. 4.

**[Table 2]**

| siRNA | Target Sequence | Sequence Number |
|---|---|---|
| siZIC5 #1 | GGCTGTGACAAATCCTACA | 5 |
| siZIC5 #2 | AAGATTCGAGGCTGTGACAAA | 6 |
| siNeg #1 | ATCCGCGCGATAGTACGTA | 7 |
| siNeg #2 | AATTCTCCGAACGTGTCACGT | 8 |

Unlike in melanoma and the like, the cell proliferation in pancreatic cancer and bile duct cancer is also controlled by not only signal transduction mediated by PDGFD but also signal transduction mainly mediated by PDGF-BB. However, as shown in FIG. 4, apoptosis was induced in the pancreatic cancer cells and the bile duct cancer cells, in which the expression of the ZIC5 gene was suppressed. Cell death occurred in 17% of the AsPC-1 line, 30% of the PANC-1 line, and 23% of the Mia Paca-2 line, which are pancreatic cancer cells, and 29% of the RBE line, which is a bile duct cancer cell. From these results, it was found that in a case of suppressing the expression of the ZIC5 gene, apoptosis is induced in the pancreatic cancer cells and the bile duct cancer cells, and the cell proliferation is suppressed.

Next, each kind of cell was introduced by transfection with siNeg and siZIC5 listed in Table 2 and cultured for 24 hours. Then, gemcitabine, which is a standard therapeutic drug for pancreatic cancer and bile duct cancer, was added thereto, the culture was further carried out for 48 hours, and then the apoptosis induction rate (%) was investigated. The results are shown in FIG. 4. As a result, apoptosis was induced by the gemcitabine (0.1 µM) treatment; however, the apoptosis induction rate was further increased in cells in which the expression of the ZIC5 gene had been suppressed. From these results, it was conceived that the ZIC5 protein promotes survival and drug resistance (primary resistance) in pancreatic cancer and bile duct cancer.

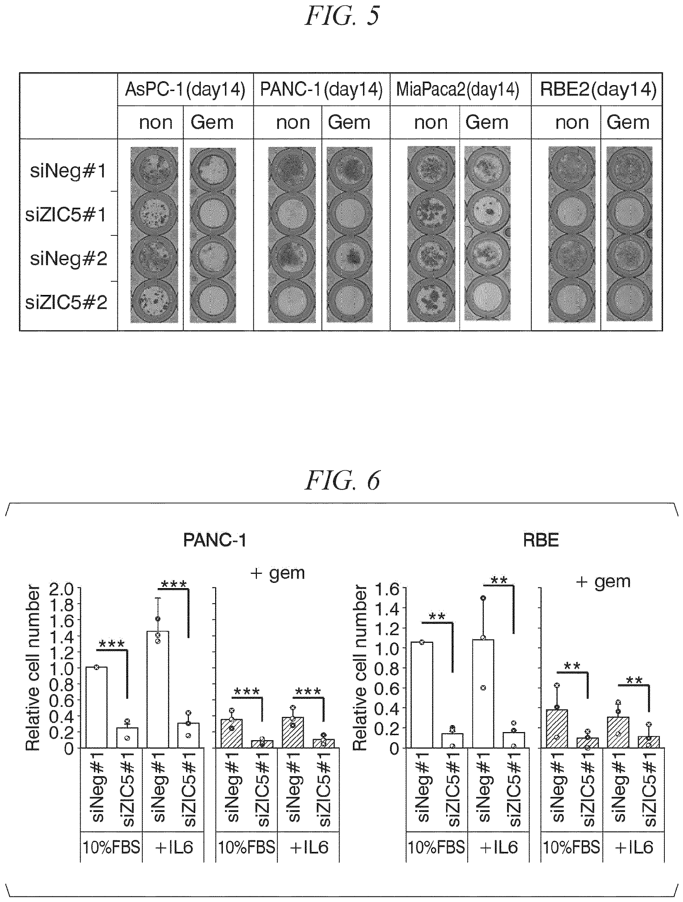

Further, in order to observe a longer-term effect, cells were introduced by transfection with siNeg and siZIC5 and cultured for 24 hours. Then, gemcitabine (0.1 µM) was added thereto, the culture was further carried out for 14 days, and then living cells were detected by Giemsa staining. FIG. 5 shows staining images. As shown in FIG. 5, it was found that regarding the PANC-1 line and the RBE line, living cells are not detected in cells transfected with siZIC5 even in a case where the gemcitabine treatment is not carried out, and cells are killed only by the suppression of the expression of the ZIC5 gene. In addition, regarding the AsPC-1 line and the MIA-PaCa-2 line, living cells were detected in cells that had not been treated with gemcitabine among the cells into which siZIC5 has been introduced; however, it was revealed that living cells are not detected in a case where the genomic drug has been treated, and cells are killed by combining the suppression of the expression of the ZIC5 gene and the genomic treatment. These results show that in pancreatic cancer and bile duct cancer, the suppression of the expression of the ZIC5 gene, that is, the reduction of the amount of the intracellular ZIC5 protein is effective for suppressing the survival of cancer cells or inhibiting the drug resistance thereof.

### [Example 2]

It was verified whether the effects of inducing the apoptosis and suppressing the proliferation of pancreatic cancer and bile duct cancer could be obtained by the reduction of the amount of the intracellular ZIC5 protein, even in a case where inflammation occurred in cancer cells and inflammatory cytokines acted on the cancer cells.

Specifically, a PANC-1 line derived from pancreatic cancer and an RBE line derived from bile duct cancer were transfected with siNeg #1 or siZIC5 #1 and subjected to the addition of IL-6 (20 ng/mL) and gemcitabine (0.1 µM) 2 days later, the culture was further continued, and the number of cells 6 days after transfection was quantified. The number of cells in a case where siNeg #1 was introduced and culture was carried out with the addition of neither IL-6 nor gemcitabine (that is, culturing in a 10% FBS medium) was set to 1, the relative cell number was measured, and the Tukey multiple comparison test was used to examine the presence or absence of the significant difference (n = 3, *p < 0.05, ***p < 0.001).

FIG. 6 shows the relative cell number under each culture condition. The left figure in FIG. 6 shows the results obtained from the PANC-1 line, and the right figure shows the results obtained from the RBE line. As shown in FIG. 6, similarly in the presence of 10% serum, cell proliferation was suppressed by suppressing the expression of the ZIC5 gene even in the presence of IL-6. A decrease in the number of cells, when treated with gemcitabine treatment, was also caused in the same manner. From these results, it was suggested that the suppression of the expression of the ZIC5 gene is effective for suppressing the cell proliferation of pancreatic cancer and bile duct cancer even in the inflammatory environment.

Next, the PANC-1 line and the RBE line were transfected with siNeg #1 or siZIC5 #1, and after 2 days, the culture medium was changed to a culture medium containing 1% FBS, a culture medium containing 1% FBS and IL-22 (20 ng/mL), or a culture medium containing 1% FBS and IL-6 (20 ng/mL), and then the culture was further carried out for 24 hours. The cells after being cultured were recovered, and Western blotting was carried out to detect phosphorylated STAT3 (pSTAT3:Tyr705), total STAT3 (STAT3), and GAPDH (a loading control). The Western blotting was carried out using an anti-pSTAT3 antibody (manufactured by Cell Signaling Technology Inc.), a STAT3 antibody (manufactured by BD Biosciences), and an anti-GAPDH antibody (manufactured by Cell Signaling Technology Inc.).

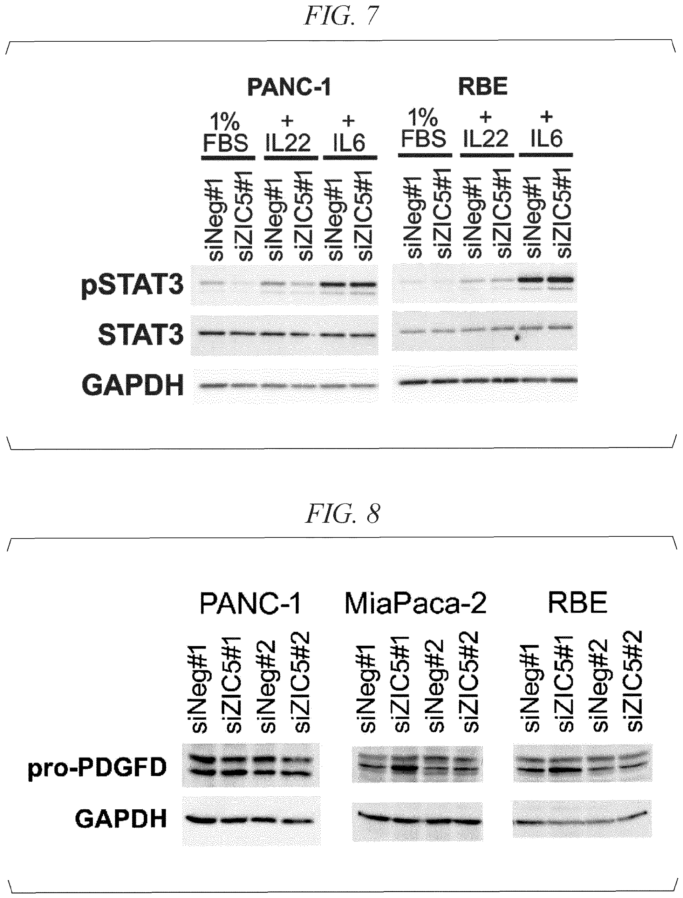

FIG. 7 shows staining images obtained from Western blotting. As shown in FIG. 7, even in a case where the expression of the ZIC5 gene was suppressed in the presence of IL-6, the phosphorylation of STAT3 was not suppressed. From these results, it was conceived that in cancer cells of pancreatic cancer and bile duct cancer, the reduction of the ZIC5 protein contributes to the suppression of proliferation even in a pathway not mediated by STAT3.

Next, the effect of suppressing the expression of the ZIC5 gene on the expression of the PDGFD gene was investigated in pancreatic cancer cells and bile duct cancer cells. Specifically, a PANC-1 line derived from pancreatic cancer, a MiaPaca-2 line derived from pancreatic cancer, and an RBE line derived from bile duct cancer were transfected with siNeg #1 or siZIC5 #1 and cultured for 3 days. The cells after being cultured were recovered, and Western blotting was carried out to detect PDGFD (pro-PDGFD) before being processed and GAPDH. The Western blotting was carried out using an anti-PDGFD antibody (manufactured by Santa Cruz Biotechnology, Inc.) and an anti-GAPDH antibody (manufactured by Cell Signaling Technology Inc.).

FIG. 8 shows staining images obtained from Western blotting. As shown in FIG. 8, in any cell lines, there was no difference in the expression level of pro-PDGFD between cells transfected with siZIC5 #1 and cells transfected with siNeg #1, and the suppression of the expression of the ZIC5 gene did not affect the expression of PDGFD.

In melanoma, cell proliferation is promoted mainly by signal transduction mediated by PDGFD, and in a case where the expression of the ZIC5 gene is suppressed, the expression of the PDGFD gene is suppressed, and the cell proliferation of melanoma cells is suppressed (Non-Patent Document 1 and Non-Patent Document 2). On the other hand, in pancreatic cancer cells and bile duct cancer cells, it was revealed that although cell proliferation is suppressed by suppressing the expression of the ZIC5 gene, the role of the ZIC5 protein in pancreatic cancer or bile duct cancer is due to a molecular mechanism different from that of melanoma or the like since there is no effect on the expression level of PDGFD.

### [Example 3]

In order to identify a compound that reduces the amount of the ZIC5 protein in cells, Validated Compound Library (about 3,500 compounds) provided by the Drug Discovery Initiative, University of Tokyo (https://www.ddi.u-tokyo.ac.jp/chemical_library/) was subjected to screening.

In the primary screening, A375 cells, derived from melanoma overexpressing a GFP-fused ZIC5 protein, were used. The cells were subjected to the addition of a candidate compound and cultured for 1 day, and then the amount of GFP fluorescence in each kind of cell was quantified using an imaging cytometer "In Cell Analyzer 2000" (manufactured by Cytiva), and the amount of GFP-ZIC5 in each kind of cell was quantified. Candidate compounds in which in the assay carried out twice, the amount of GFP fluorescence was equal to or less than "the average value of the amount of GFP fluorescence in control cells - 3 SD" were selected as primary hit compounds.

In the secondary screening, A375 cells overexpressing the ZIC5 protein (without a fusion protein) were subjected to the addition of the primary hit compound to a final concentration of 0.1 µM, 1 µM, 5 µM, or 10 µM and cultured, and then the cells were fixed on the next day. The fixed cells were subjected to staining by using an anti-ZIC5 antibody (clone: GTX104840) and an Alexa Fluor 568-anti-rabbit IgG antibody as a secondary antibody, and the amount of the ZIC5 protein was quantified using the imaging cytometer. 12 kinds of primary hit compounds (compounds (T-1) to (T-12)) were selected as ZIC5 degradation promoting compounds, where the primary hit compounds gave an average fluorescence intensity smaller than "the average value from cells untreated with the compound - SD" in the two assays.

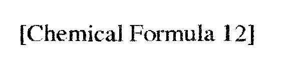

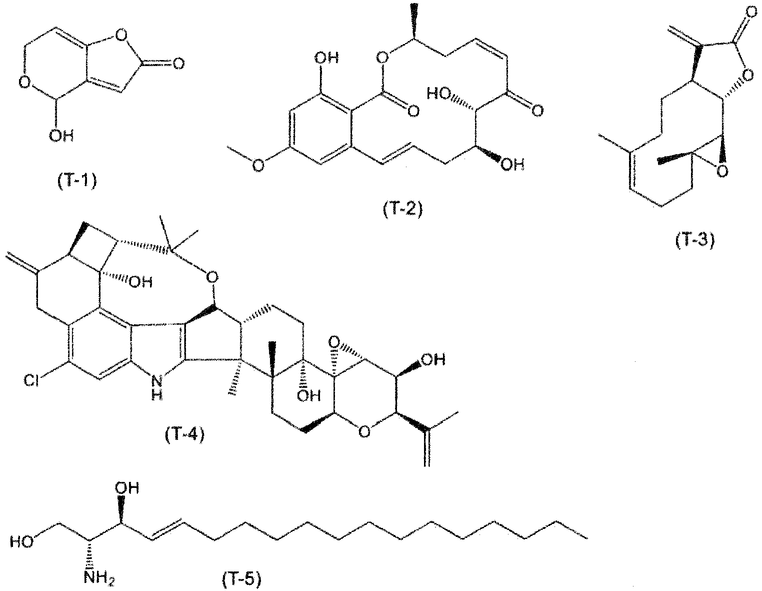

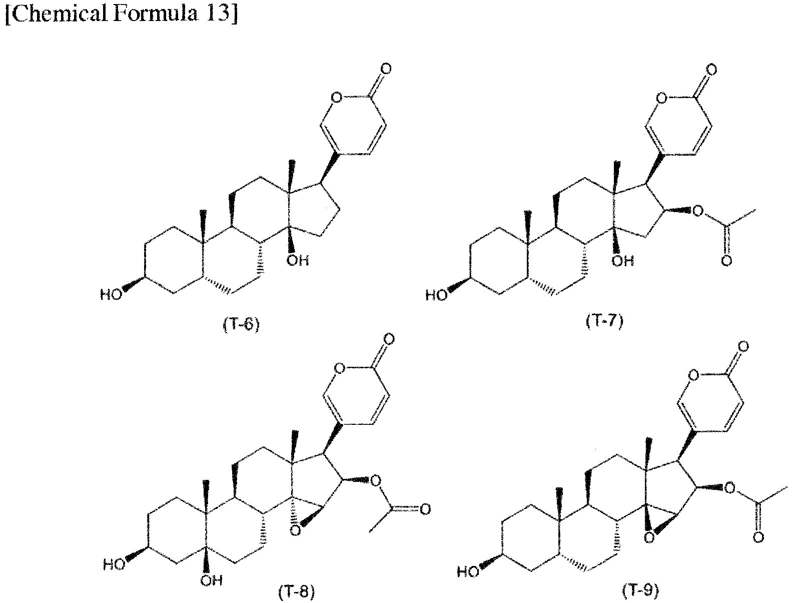

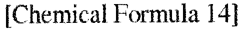

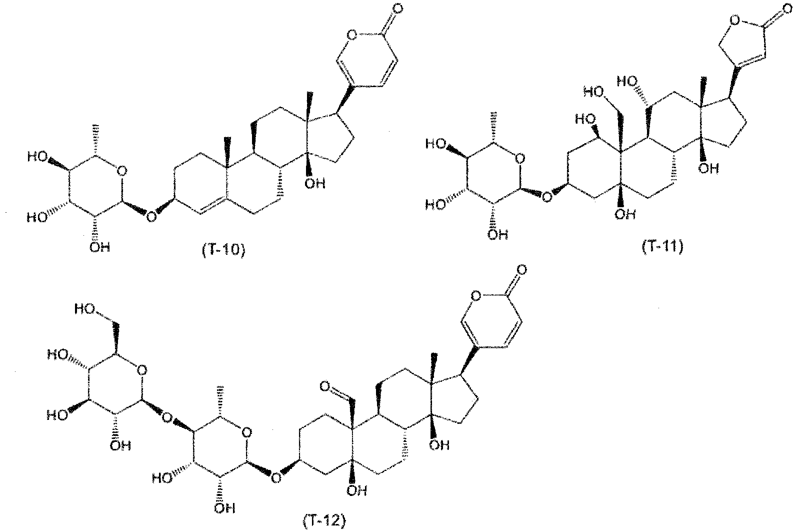

Table 3 shows the relative amount of the ZIC5 protein in a case where the amount of the ZIC5 protein in control cells (cells untreated with the compound) is set to 100. In the table, "ND" indicates a sample in which measurement was impossible due to cell death. The numerical value of the mean value - SD is 78.6.

**[Table 3]**

| | ZIC5 Protein Amount (Relative Value) | | | |
|---|---|---|---|---|
| Compound | 10 µM | 5 µM | 1 µM | 0.1 µM |
| T-1 | ND | ND | 76.69 | 83.38 |
| T-2 | 70.79 | 72.79 | 83.09 | 89.63 |
| T-3 | ND | 55.29 | 98.17 | 89.82 |
| T-4 | ND | 75.42 | 96.02 | 111.04 |
| T-5 | ND | 62.57 | 118.36 | 107.66 |
| T-6 | ND | ND | 45.86 | 91.86 |
| T-7 | ND | ND | 76.36 | 103.20 |
| T-8 | 44.74 | 70.55 | 90.80 | 88.24 |
| T-9 | ND | 77.07 | 157.11 | 102.67 |
| T-10 | ND | 49.98 | 42.12 | 70.25 |
| T-11 | ND | ND | 67.77 | 90.59 |
| T-12 | ND | 57.15 | 72.50 | 76.80 |

As shown in Table 3, all thereof could significantly reduce the amount of the intracellular ZIC5 protein within any range of 0.1 to 10 µM, as compared with the control cells. That is, it was confirmed that these 12 kinds of compounds are ZIC5 protein reducing substances.

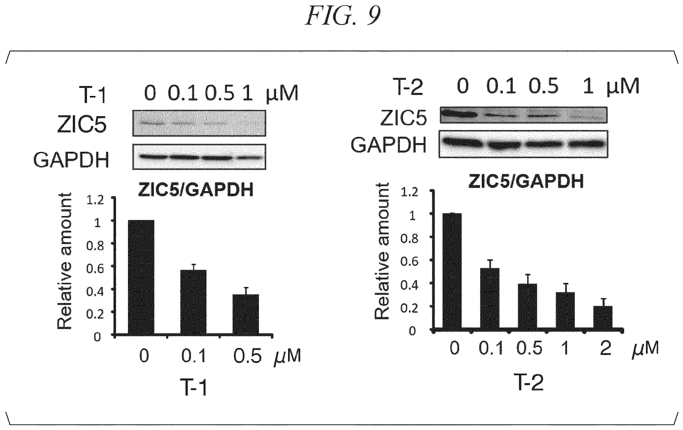

Regarding the compound (T-1) and the compound (T-2), the effect on the amount of the intracellular ZIC5 protein was investigated according to the Western blotting method. Specifically, A375 cells (none was overexpressed therein) were subjected to the addition of the compound (T-1) or the compound (T-2) so that the concentration thereof was 0, 0.1, 0.5, or 1 µM, and the cells were cultured for 48 hours. The cells after being cultured were recovered, and Western blotting was carried out using an anti-ZIC5 antibody and an anti-GAPDH antibody. The amount of each protein was quantified from a density of a band in the Western blotting image. FIG. 9 shows the relative amount of the ZIC5 protein ([amount of ZIC5 protein]/[amount of GAPDH protein]) together with the Western blotting images. As shown in FIG. 9, it was confirmed that the amount of the ZIC5 protein in cells is reduced depending on the concentrations of the compound (T-1) and the compound (T-2), and thus these compounds have the action of reducing the amount of the ZIC5 protein in cells.

### [Example 4]

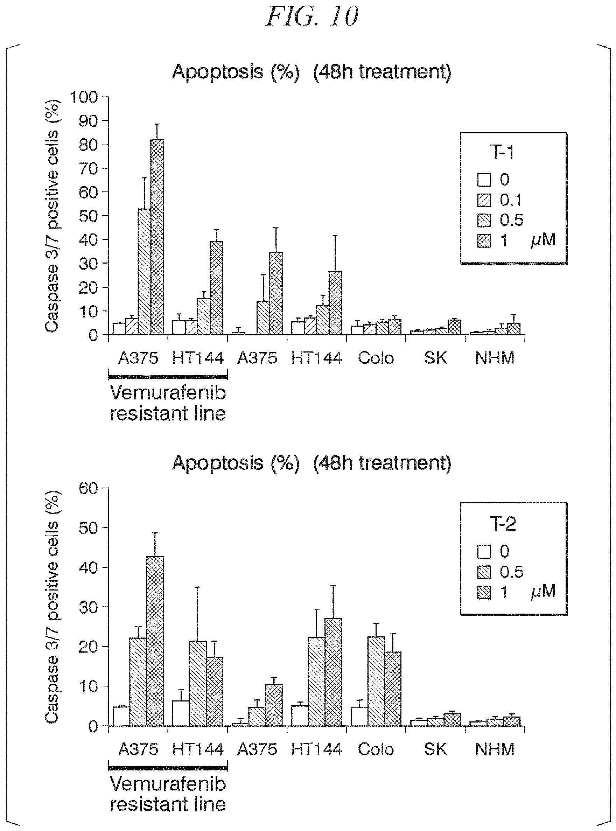

A total of 7 kinds of cells of 2 kinds of vemurafenib-resistant lines derived from human melanoma (an A375vemR line and an HT144vemR line), 2 kinds of ZIC5 high-expressing melanoma cell lines (an A375 line and an HT144 line), 2 kinds of ZIC5 low-expressing melanoma cell lines (a Colo829 line and an SKmel28 line), and a ZIC5 non-expressing normal human melanocyte cell (NHM) were treated with the compound (T-1) or compound (T-2), and regarding the cells that had been subsequently cultured for 48 hours, the apoptosis induction rate (%) was determined in the same manner as in Example 1. The compound (T-1) was treated so that the concentration thereof was 0, 0.1, 0.5, or 1 µM, and the compound (T-2) was treated so that the concentration thereof was 0, 0.1, or 1 µM, FIG. 10 shows the measurement results. The upper part of FIG. 10 shows the results obtained from the cells treated with the compound (T-1), and the lower part thereof shows the results obtained from the cells treated with the compound (T-2).

The compound (T-1) induced apoptosis in the vemurafenib-resistant lines derived from human melanoma and the ZIC5 high-expressing melanoma cell lines at a concentration of 1.2 µM, whereas it did not induce apoptosis in the ZIC5 low-expressing melanoma cell lines and the ZIC5 non-expressing normal human melanocyte cell. On the other hand, the compound (T-2) induced apoptosis in one kind (the Colo829 line) among the vemurafenib-resistant lines derived from human melanoma, the ZIC5 high-expressing melanoma cell lines, and the ZIC5 low-expressing melanoma cell lines at a concentration of 1.2 µM, whereas it did not induce apoptosis in one kind (the SKmel28 line) of the ZIC5 low-expressing melanoma cell lines and the ZIC5 non-expressing normal human melanocyte cell. From the above results, it was shown that the compound (T-1) and the compound (T-2) tend to induce apoptosis in cancer cell lines having high expression of ZIC5.

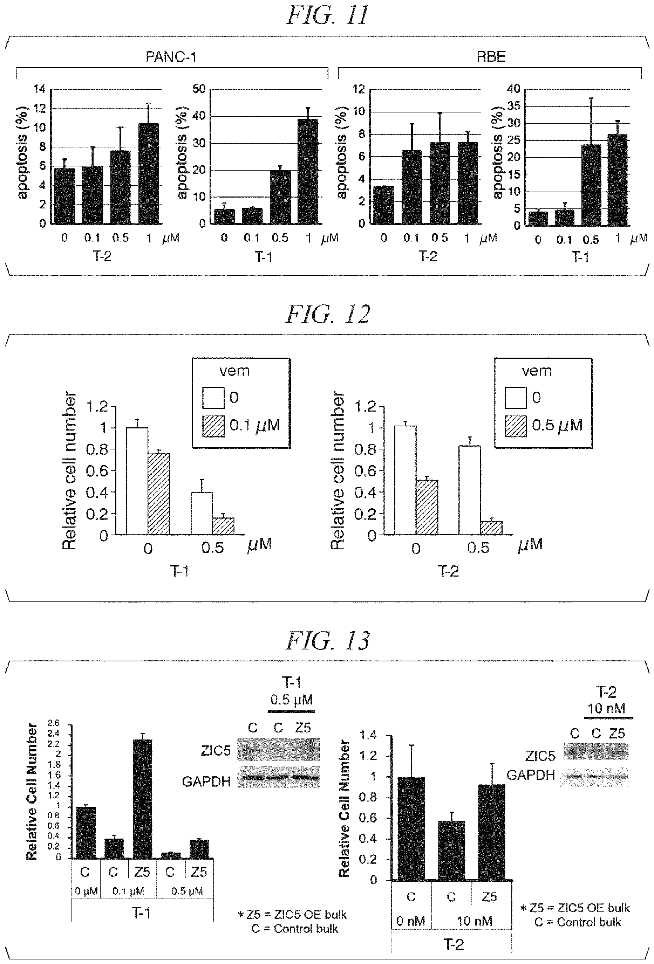

Next, the pancreatic cancer cell line (the Panc-1 line) and the bile duct cancer cell line (the RBE line) were treated with the compound (T-1) or compound (T-2) in the same manner, and regarding the cells that had been subsequently cultured for 48 hours, the apoptosis induction rate (%) was determined in the same manner as in Example 1. FIG. 11 shows the measurement results. Regarding the data of each kind of cell line, the right figure shows the results obtained from the cells treated with the compound (T-1), and the left figure shows the results obtained from the cells treated with the compound (T-2). As shown in FIG. 11, both the compound (T-1) and the compound (T-2) induced apoptosis in the pancreatic cancer cell line (the Panc-1 line) and the bile duct cancer cell line (the RBE line). In particular, the apoptosis induction rate of the compound (T-2) was about 8% to 10%, whereas the compound (T-1) induced apoptosis at a rate of 20% to 40%.

### [Example 5]

It was verified whether the ZIC5 protein reducing substance changes the sensitivity to BRAF inhibitors (PLX4032 and vemurafenib) (manufactured by Selleck Chemicals LLC).

Specifically, 0.5 µM of the compound (T-1) or 50 nM of the compound (T-2) was added to A375 cells, culturing was carried out for 48 hours, and then vemurafenib was added thereto a concentration of 0.1 µM, and culturing was further carried out for 72 hours. After the culturing, cell nuclei were stained with Hoechst to count the number of cells. The number of cells of the A375 cells cultured without adding any compound was set to 1, and the relative cell number of each kind of cell was determined. FIG. 12 shows the results. In FIG. 12, the left figure shows the results obtained from the cells treated with the compound (T-1), and the right figure shows the results obtained from the cells treated with the compound (T-2). As a result, it was confirmed that the compound (T-1) of compound (T-2), and vemurafenib synergistically reduce the number of cells, that is, synergistically suppress cell proliferation.

### [Example 6]

It was verified whether or not the apoptosis inducing effect of the compound (T-1) and the compound (T-2) is generated by reducing the amount of the ZIC5 protein.

Specifically, a ZIC5 expression plasmid (Z5) or an empty vector (C) was introduced into A375 melanoma cells by transfection, and cells into which each of the plasmids had been introduced were selected. Next, these cells were subjected to the addition of 0.1 µM or 0.5 µM of the compound (T-1) or 10 nM of the compound (T-2) and cultured for 4 days. After the culturing, cell nuclei were stained with Hoechst, and the number of cells was counted with an imaging cytometer "IN Cell Analyzer 2000". The number of cells, into which an empty vector had been introduced and which had not been subjected to the compound treatment, was set to 1, and the relative cell number of each kind of cell was determined. FIG. 13 shows the results together with the staining images obtained from Western blotting. In FIG. 13, the left figure shows the results obtained from the cells treated with the compound (T-1), and the right figure shows the results obtained from the cells treated with the compound (T-2). In the figure, "C" is for cells into which the empty vector has been introduced, and "Z5" is for cells into which the ZIC5 expression plasmid has been introduced.

As shown in FIG. 13, in the cells (Z5) in which the amount of the ZIC5 protein was compensated by ZIC5 overexpression, the apoptosis induction and the suppression of the cancer cell proliferation by the compound (T-1) and the compound (T-2) were restored. From these results, it was shown that the effect of suppressing the cell proliferation of cancer cells by these ZIC5 protein reducing substances is partially caused by a decrease in the amount of ZIC5 protein in cells.

### [Example 7]

It was investigated whether or not a small hairpin RNA (shRNA) targeting ZIC5 suppresses the proliferation of cancer cells. shZIC5 listed in Table 4 was used as an shRNA targeting ZIC5, and shNeg was used as a negative-control shRNA.

**[Table 4]**

| siRNA | Target Sequence | Sequence Number |
|---|---|---|
| shZIC5 | GGCTGTGACAAATCCTACA | 9 |
| shNeg | GTGATGAATCCAGCCTACA | 10 |

First, PlatA cells for retrovirus production were transfected with shZIC5/pSIREN-GFP obtained by introducing an shRNA (shZIC5) that suppresses ZIC5 into a pSIREN-RetroQ-ZsGreen vector (manufactured by Clontech Laboratories, Inc.), and shNeg/pSIREN obtained by introducing a negative control shRNA (shNeg) into a pSlREN-RetroQ-ZsGreen vector, whereby retrovirus was produced. Pancreatic cancer cells (a MiaPaca-2 line) were infected with the recovered retrovirus to introduce ZIC5/pSIREN-GFP or shNeg/pSIREN-GFP into the pancreatic cancer cells. For a proliferation assay, the pancreatic cancer cells after the retrovirus infection were seeded in a 96-well plate and cultured for 3 days, and then the number of GFP-positive cells was measured to determine the proliferation rate ([number of GFP-positive cells on day 3 of culture]/[number of GFP-positive cells on day 0 of culture]).

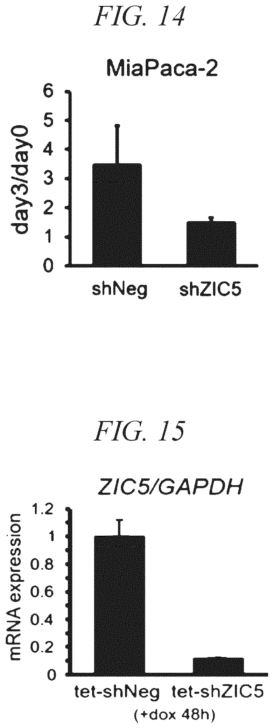

FIG. 14 shows the measurement results of the proliferation rate of pancreatic cancer cells on day 3 of the culture after virus infection. As compared with pancreatic cancer cells into which shNeg was introduced, a decrease in proliferation rate was also observed in pancreatic cancer cells into which shZIC5/pSIREN-GFP that suppresses ZIC5 was introduced.

### [Example 8]

It was investigated whether the suppression of ZIC5 exhibits a tumor regression effect on tumors engrafted in vivo, by using a mouse transplanted with cancer cells capable of inducing the suppression of the expression of ZIC5 by the tet-on system.

First, A vector (tet-shZIC5) obtained by carrying out insertion of the shZIC5 used in Example 7 and a vector (tet-shNeg) obtained by carrying out insertion of the shNeg in the same manner were constructed in a plasmid vector (Tet-pLKO-puro, manufactured by Addgene) capable of inducing expression of shRNA by the tet-on system.

Tet-shZIC5 or tet-shNeg was introduced into human pancreatic cancer cells (a MiaPaca-2 line) by viral infection, thereby producing pancreatic cancer cells (tet-shZIC5-MiaPaca-2) capable of inducing the expression of shZIC5 by the tet-on system, and pancreatic cancer cells (tet-shNeg-MiaPaca-2) capable of inducing the expression of shNeg by the tet-on system.

It was checked whether the expression of each shRNA was induced in the MiaPaca-2 cells into which each tet-shRNA had been introduced, by the addition of doxycycline (dox) which is a tetracycline (tet) derivative. First, dox was added to the culture medium of each kind of cell, and the cells were cultured for 48 hours. The mRNA level of the ZIC5 gene in the cells after being cultured was measured using the amount of the mRNA of the GAPDH gene as an internal standard. The measurement of mRNA was carried out by qRT-PCR in the same manner as in Reference Example 2.

FIG. 15 shows the measurement results of the amount of the mRNA of the ZIC5 gene in each kind of cell. In the tet-shZIC5-MiaPaca-2 cells, the dox treatment for 48 hours reduced the amount of the mRNA of the ZIC5 gene to about 10% of that in the control tet-shNeg-MiaPaca-2 cells.

Subsequently, the tet-shZIC5-MiaPaca-2 cells or the tet-shNeg-MiaPaca-2 cells were subcutaneously transplanted into and engrafted into immunodeficient mice (Balb-c nu/nu) (n = 3 in each case). On the 23rd day after the transplantation, it was confirmed that the volume of the tumor was about 100 mm3, and then dox was added to the potable water of all the mice until the end of the experiment (the 37th day after the transplantation), whereby a dox treatment was carried out. From 10 days after the addition of dox, all the mice were intraperitoneally administered with gemcitabine (100 mg/kg body weight) once a day for a total of 3 days.

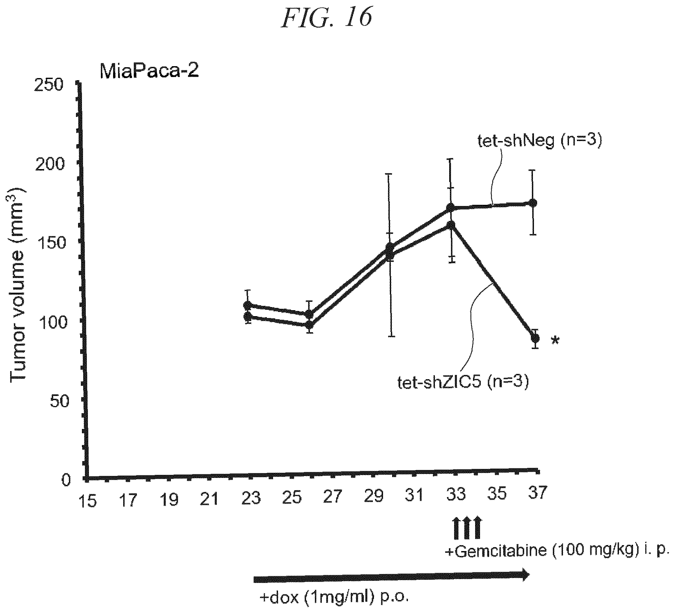

FIG. 16 shows the results obtained by time-dependently measuring the tumor volume during a period from the 23rd day to the 37th day after the transplantation. As shown in FIG. 16, as compared with the tumor into which the control tet-shNeg-MiaPaca-2 cells were transplanted, a reduction of the tumor volume was observed after the administration of gemcitabine in the tumors into which the tet-shZIC5-MiaPaca-2 cells were transplanted. From these results, it was conceived that in the human pancreatic cancer cells engrafted into the mouse living body, the suppression of ZIC5 enhances the sensitivity to gemcitabine, and thus a synergistic effect has been obtained.

## Claims

1. A pharmaceutical composition comprising, as an active ingredient:
a substance that reduces an intracellular content of a ZIC5 protein,
wherein the composition is used for treatment of pancreatic cancer or bile duct cancer.

2. The pharmaceutical composition according to Claim 1,
wherein the substance that reduces the intracellular content of the ZIC5 protein is a substance that suppresses expression of a ZIC5 gene.

3. The pharmaceutical composition according to Claim 2,
wherein the substance that suppresses the expression of the ZIC5 gene is a nucleic acid molecule that suppresses the expression of the ZIC5 gene by RNA interference.

4. The pharmaceutical composition according to Claim 1,
wherein the substance that reduces the intracellular content of the ZIC5 protein is any compound represented by General Formula (1) or a derivative thereof, a salt thereof, or a solvate thereof 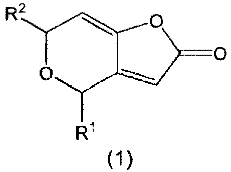 [in the formula, R¹ and R² are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

5. The pharmaceutical composition according to Claim 1,
wherein the substance that reduces the intracellular content of the ZIC5 protein is any compound represented by General Formula (2) or a derivative thereof, a salt thereof, or a solvate thereof. 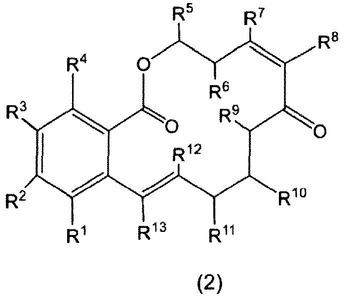 [in the formula, R¹ to R¹³ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

6. The pharmaceutical composition according to Claim 1,
wherein the substance that reduces the intracellular content of the ZIC5 protein is any compound represented by General Formula (3) or a derivative thereof, a salt thereof, or a solvate thereof 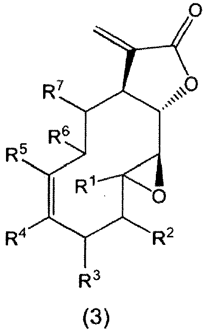 [in the formula, R¹ to R⁷ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

7. The pharmaceutical composition according to Claim 1,
wherein the substance that reduces the intracellular content of the ZIC5 protein is any compound represented by General Formula (4) or a derivative thereof, a salt thereof, or a solvate thereof. 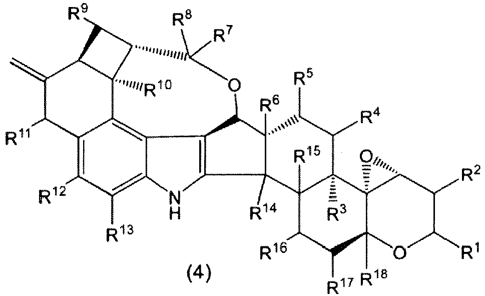 [in the formula, R¹ to R¹⁸ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

8. The pharmaceutical composition according to Claim 1,
wherein the substance that reduces the intracellular content of the ZIC5 protein is any compound represented by General Formula (5) or a derivative thereof, a salt thereof, or a solvate thereof 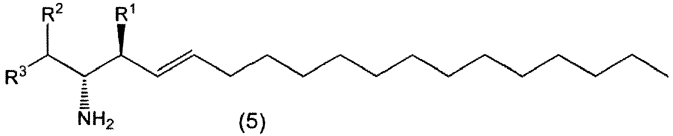 [in the formula, R¹ to R³ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

9. The pharmaceutical composition according to Claim 1,
wherein the substance that reduces the intracellular content of the ZIC5 protein is any compound represented by General Formula (6) or a derivative thereof, a salt thereof, or a solvate thereof 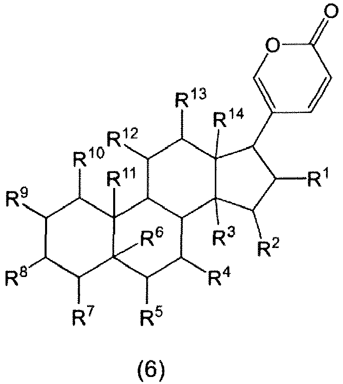 [in the formula, R¹ to R¹⁴ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

10. The pharmaceutical composition according to Claim 1,
wherein the substance that reduces the intracellular content of the ZIC5 protein is any compound represented by General Formula (7) or a derivative thereof, a salt thereof, or a solvate thereof 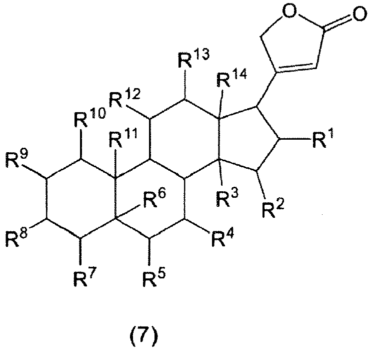 [in the formula, R¹ to R¹⁴ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

11. The pharmaceutical composition according to Claim 1,
wherein the substance that reduces the intracellular content of the ZIC5 protein is any compound represented by General Formula (8) or a derivative thereof, a salt thereof, or a solvate thereof 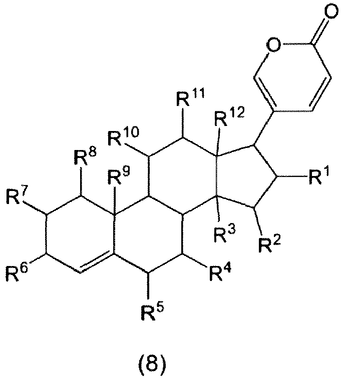 [in the formula, R¹ to R¹² are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

12. The pharmaceutical composition according to Claim 1,
wherein the substance that reduces the intracellular content of the ZIC5 protein is any compound represented by General Formula (9) or a derivative thereof, a salt thereof, or a solvate thereof 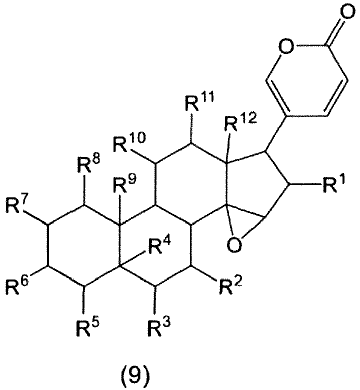 [in the formula, R¹ to R¹² are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].

13. The pharmaceutical composition according to any one of Claims 1 to 12,
wherein the pancreatic cancer or the bile duct cancer is a cancer in which the ZIC5 protein is expressed.

14. The pharmaceutical composition according to any one of Claims 1 to 12, further comprising:
an anti-cancer agent other than the substance that reduces the intracellular content of the ZIC5 protein.

15. The pharmaceutical composition according to any one of Claims 1 to 12, further comprising:
one or more anti-cancer agents selected from the group consisting of a BRAF inhibitor and a DNA synthesis inhibitor.

16. A ZIC5 protein reducing agent comprising, as an active ingredient:
a compound represented by any of General Formulae (1) to (9) or a derivative thereof, a salt thereof, or a solvate thereof 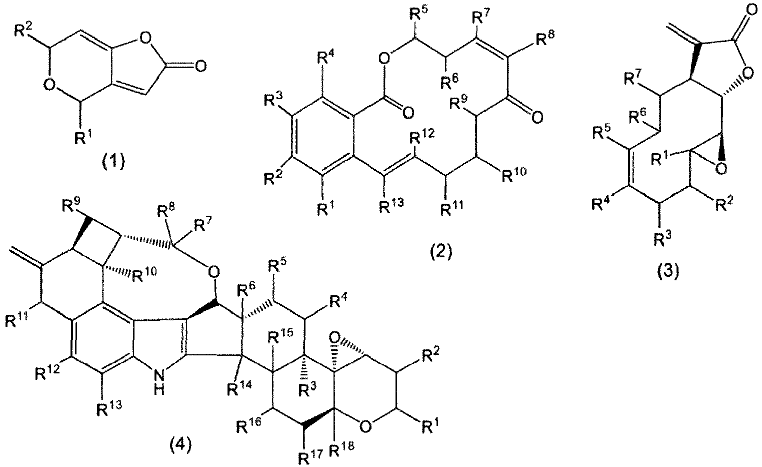 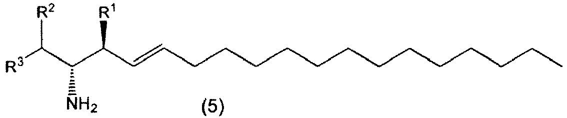 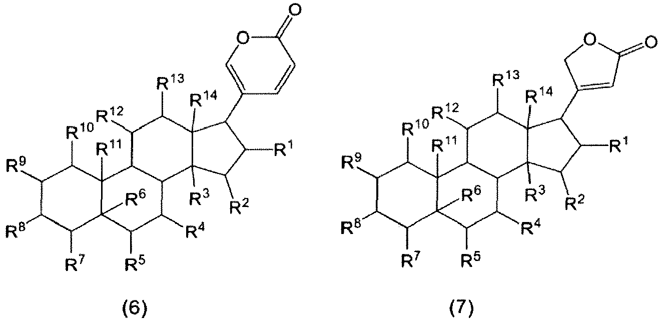 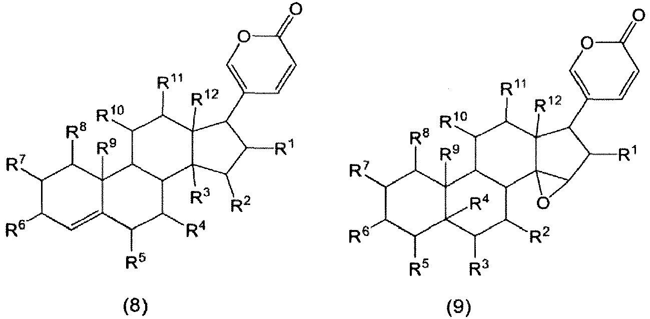 [in the formulae, R¹ to R¹⁸ are each independently a hydrogen atom, a halogen atom, a hydroxy group, an alkyl group having 1 to 6 carbon atoms, an alkenyl group having 2 to 6 carbon atoms, a hydroxyalkyl group, an alkoxy group, a formyl group, an acyloxy group, or a tetrahydropyranyloxy group which may have a substituent].
